# FASCICULE DE BREVET EUROPEEN

(11) **EP 1 462 445 B1**
(45) Date de publication et mention de la délivrance du brevet: **18.10.2006**
(21) Numéro de dépôt: 04290723.8
(22) Date de dépôt: 17.03.2004
(51) Int. Cl.: C07D 233/54, C07D 243/06, C07D 295/12, C07D 403/12, A61Q 5/10

(54) **Dérivés de paraphénylènediamine à groupement cyclique diaza substitué par un radical cationique et utilisation de ces dérivés pour la coloration de fibres kératiniques**
Paraphenylendiamin Derivate auf Basis einer zyklischen Diaza-Verbindung substituiert mit einem kationischen Radikal und Verwendung dieser Derivate zur Färbung von Keratinfasern
Paraphenyldiamine derivatives concisting of a cyclic diaza group substituted by a cationic radical and use of these derivatives for dyeing keratin fibres

(30) Priorité: 24.03.2003 FR 0303548
(43) Date de publication de la demande: 29.09.2004
(73) Titulaire: L'ORÉAL, 75008 Paris (FR)
(72) Inventeur: Sabelle, Stéphane, 75005 Paris (FR); Genet, Alain, 93600 Aulnay sous Bois (FR); Leduc, Madeleine, 29, Rue des Boulets 75011 Paris (FR)
(74) Mandataire: Fevrier, Murielle Françoise E.

(56) Documents cités:
- EP-A- 0 891 765
- EP-A- 0 962 452
- EP-A- 0 966 449
- WO-A-03/014093
- PATENT ABSTRACTS OF JAPAN vol. 1999, no. 11, 30 septembre 1999 (1999-09-30) & JP 11 158048 A (FUJI PHOTO FILM CO LTD), 15 juin 1999 (1999-06-15)

## Description

L'invention a pour objet de nouveaux dérivés de paraphénylènediamine à groupement cyclique diaza substitué par un radical cationique, les compositions tinctoriales les contenant ainsi que le procédé de teinture de fibres kératiniques à partir de ces compositions.

Il est connu de teindre les fibres kératiniques et en particulier les cheveux humains avec des compositions tinctoriales contenant des précurseurs de colorant d'oxydation, en particulier des ortho ou para-phénylènediamines, des ortho ou para-aminophénols, des composés hétérocycliques tels que des dérivés de diaminopyrazole, des dérivés de pyrazolo[1,5-a]pyrimidine, des dérivés de pyrimidines, des dérivés de pyridine, des dérivés de 5,6-dihydroxyindole, des dérivés de 5,6-dihydroxyindoline appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-hydroxyphénols et certains composés hétérocycliques tels que par exemple des dérivés de pyrazolo[1,5-b]-1,2,4,-triazoles, des dérivés de pyrazolo[3,2-c]-1,2,4,-triazoles, des dérivés de pyrazolo[1,5-a]pyrimidines, des dérivés de pyridine, des dérivés de pyrazol-5-one, des dérivés d'indoline et des dérivés d'indole.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée, présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possibles, c'est à dire permettre d'obtenir des écarts de coloration les plus faibles possibles tout au long d'une même fibre kératinique, qui peut être en effet différemment sensibilisée (i.e. abîmée) entre sa pointe et sa racine. Ils doivent également présenter une bonne stabilité chimique dans les formulations. Ils doivent présenter un bon profil toxicologique.

Dans le domaine de la coloration capillaire, la para-phénylènediamine, la para-toluènediamine sont des bases d'oxydation largement utilisées. Elles permettent d'obtenir avec des coupleurs d'oxydation des nuances variées.

Cependant, il existe un besoin de découvrir de nouvelles base d'oxydation présentant un meilleur profil toxicologique que la para-phénylènediamine et la para-toluènediamine, tout en permettant de conférer aux cheveux d'excellentes propriétés d'intensité de couleur, de variété de nuances, d'uniformité de la couleur et de ténacité aux agents extérieurs.

Il est déjà connu d'utiliser des dérivés de paraphénylènediamine subtitués par un groupement pyrrolidinique comme base d'oxydation pour la coloration de fibres kératiniques afin de remplacer la para-phénylènediamine. Par exemple, le brevet US 5,851,237 décrit l'utilisation de dérivés 1-(4-aminophényl)pyrrolidine éventuellement substitués sur le noyau benzénique. Le brevet US 5,993,491 propose l'utilisation de dérivés de N-(4-aminophényl)-2-hydroxyméthylpyrrolidine éventuellement substitués sur le noyau benzénique et sur l'hétérocycle pyrrolidinique en position 4 par un radical hydroxy.

La demande de brevet JP 11-158048 propose des compositions contenant au moins un composé choisi parmi des dérivés de paraphénylènediamine éventuellement substitués sur le noyau benzénique et dont un des atomes d'azote est compris dans un cycle de 5 à 7 chaînons carbonés.

Le document DE 19707545 décrit l'utilisation de composés paraphénylènediamine dont un des groupes amino forme un diazacycloheptane pour la teinture des fibres kératiniques.

Cependant, ces composés ne permettent pas de conférer aux cheveux une coloration de qualité équivalente à celle obtenue avec la para-phénylènediamine ou avec la para-toluènediamine du fait d'un manque d'intensité et d'uniformité de la couleur.

Le but de la présente invention est de développer de nouvelles compositions tinctoriales ne présentant pas les inconvénients des bases d'oxydation de la technique antérieure. En particulier, le but de l'invention est de fournir de nouvelles bases d'oxydation présentant à la fois un bon profil toxicologique et des propriétés telles que les compositions tinctoriales les contenant ne dégradent pas les fibres kératiniques tout en étant capables d'engendrer des colorations intenses dans des nuances variées, peu sélectives et particulièrement résistantes.

Ce but est atteint avec la présente invention qui a pour objet un dérivé de paraphénylènediamine substitué par un groupement cyclique diaza de formule (I) et ainsi que les sels d'addition correspondants 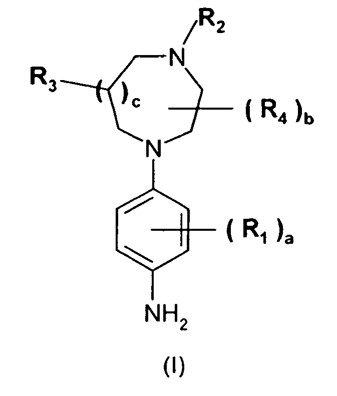 dans laquelle
- **a** est compris entre 0 et 4, étant entendu que lorsque a est supérieur ou égal à 2 alors les radicaux R₁ peuvent être identiques ou différents,
- **b** est compris entre 0 et 4, étant entendu que lorsque b est supérieur ou égal à 2 alors les radicaux R₄ peuvent être identiques ou différents,
- **c** est 0 ou 1,
- **R**_{**1**} représente un atome d'halogène ; une chaîne hydrocarbonée en C₁-C₈, aliphatique ou alicyclique, saturée ou insaturée, un ou plusieurs atomes de carbone pouvant être remplacés par un ou plusieurs atomes d'oxygène, d'azote, de silicium, de soufre ou un groupement SO₂; un radical onium Z ; le radical R₁ ne comportant pas de liaison peroxyde, ni de radicaux diazo, nitro ou nitroso ,
- **R**_{**2**} représente un radical onium Z
- **R**_{**3**} représente
   - un radical alkyle ;
   - un radical alcényle ;
   - un radical alcynyle ;
   - un radical hydroxy;
   - un radical hydroxyalkyle ;
   - un radical alcoxy ;
   - un radical alcoxyalkyle ;
   - un radical alkylcarbonyle ;
   - un radical hydroxyalcoxyalkyle ;
   - un radical amino,
   - un radical monoalkylamino ou dialkylamino ;
   - un radical amino alkyle ;
   - un radical aminoalkyle dont l'amine est mono ou disubstitué par un radical alkyle, acétyle ou hydroxyalkyle ;
   - un radical hydroxy et amino alkyle ;
   - un radical carboxyle ;
   - un radical carboxyalkyle ;
   - un radical carbamoyle ;
   - un radical carbamoylalkyle ;
   - un radical alkoxycarbonyle ;
   - un radical mono- ou dialkyl- aminocarbonyle ;
   - un radical mono- ou dialkyl- aminocarbonylalkyle ;
- **R**_{**4**} représente
   - un radical alkyle
   - un radical alcényle ;
   - un radical alcynyle ;
   - un radical hydroxyalkyle ;
   - un radical alcoxyalkyle ;
   - un radical alkylcarbonyle ;
   - un radical hydroxyalcoxyalkyle ;
   - un radical aminoalkyle ;
   - un radical aminoalkyle dont l'amine est mono ou disubstitué par un radical alkyle, acétyle, hydroxyalkyle ;
   - un radical hydroxy et amino alkyle ;
   - un radical carboxyle ;
   - un radical carboxyalkyle ;
   - un radical carbamoyle ;
   - un radical carbamoylalkyle ;
   - un radical alkoxycarbonyle ;
   - un radical mono- ou dialkyl- aminocarbonyle ;
   - un radical mono- ou dialkyl- aminocarbonylalkyle.

L'invention a aussi pour objet les dérivés nitro de formule (II) entrant dans la synthèse des dérivés de formule (I) de la présente invention.

Un autre objet de l'invention est une composition tinctoriale contenant au moins un dérivé de paraphénylènediamine de formule (I) à titre de base d'oxydation.

L'invention a aussi pour objet l'utilisation de cette composition pour la teinture de fibres kératiniques et le procédé de teinture de fibres kératiniques, en particuliers les fibres kératiniques humaines telles que les cheveux, mettant en oeuvre la composition de la présente invention.

La composition de la présente invention permet en particulier d'obtenir une coloration des fibres kératiniques chromatique, puissante, peu sélective et tenace.

Dans le cadre de l'invention, une chaîne hydrocarbonée aliphatique est une chaîne linéaire ou ramifiée pouvant contenir des insaturations du type alcène ou alcyne. Une chaîne hydrocarbonée alicyclique est une chaîne ramifiée saturée ou insaturée ne contenant pas de structure cyclique aromatique.

On entend par le terme "onium" un radical quaternaire d'une base azotée.

Les composés de formule (I) sont des para-phénylènediamines dont une amine est comprise dans un cycle de type 1,4-diazacycloheptane, cycle aussi appelé dans la littérature 1,4-diazépane, ou dont une amine est comprise dans un cycle de type 1,4-diazacyclohexane aussi appelé dans la littérature 1,4-pipérazine.

Dans la formule (I) ci-dessus, R₁ peut être choisi parmi un radical alkyle en C₁-C₄, un radical alcoxyalkyle en C₁-C₄, un radical hydroxyalkyle en C₁-C₄, un radical aminoalkyle en C₁-C₄, un radical alcoxy en C₁-C₄, un radical hydroxyalcoxy en C₁-C₄, un radical carboxyalkyle en C₁-C₄.

A titre d'exemple, on peut citer pour R1 un atome de chlore, un radical méthyle, éthyle, isopropyle, vinyle, allyle, méthoxyméthyle, 1-hydroxyéthyle, 1-carboxyméthyle, 1-aminométhyle, 2-carboxyéthyle, 2-hydroxyéthyle, 3-hydroxypropyle, 1,2-dihydroxyéthyle, 1-hydroxy-2-aminoéthyle, 1-amino-2-hydroxyéthyle, 1,2-diaminoéthyle, méthoxy, éthoxy, allyloxy, 2-hydroxyéthyloxy.

Selon un mode de réalisation particulier, R₁ peut être choisi parmi le chlore, le brome, un radical alkyle en C₁-C₄, hydroxyalkyle en C₁-C₄, aminoalkyle en C₁-C₄, alcoxy en C₁-C₄, hydroxyalcoxy en C₁-C₄. R₁ est alors de préférence choisi parmi un radical méthyle, hydroxyméthyle, 2-hydroxyéthyle, 1,2-dihydroxyéthyle, méthoxy, isopropyloxy, 2-hydroxyéthoxy.

Selon un mode de réalisation particulier, a est égal à 0 ou 1, de préférence 0.

Selon un mode de réalisation particulier, R₂ représente le radical onium Z correspondant à la formule (II) 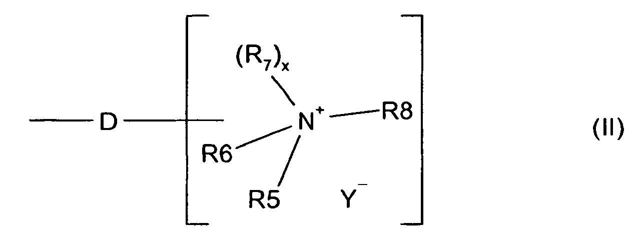 dans laquelle
- D est une liaison covalente ou une chaîne alkylène en C₁-C₁₄, linéaire ou ramifiée pouvant contenir un ou plusieurs hétéroatomes choisis parmi l'oxygène, le soufre ou l'azote, et pouvant être substituée par un ou plusieurs radicaux hydroxyle ou amino, et pouvant comprendre un ou plusieurs carbonyles;
- **R**_{**8**}**, R**_{**5**} et **R**_{**6**}**,** pris séparément, représentent un radical alkyle en C₁-C₁₅ ; un radical monohydroxyalkyle en C₁-C₆; un radical polyhydroxyalkyle en C₂-C₆ un radical alcoxy(C₁-C₆)alkyle en C₁-C₆; un radical aryle ; un radical benzyle ; un radical carbamoylalkyle en C₁-C₆ ; un radical trialkyl(C₁-C₆)silanealkyle en C₁-C₆; un radical aminoalkyle en C₁-C₆; un radical aminoalkyle en C₁-C₆ dont l'amine est mono- ou disubstituée par un radical alkyle en C₁-C₄, alkyl(C₁-C₆)carbonyle, carbamoyle ou alkyl(C₁-C₆)sulfonyle ; un radical ammonium quaternaire ;
- **R**_{**8**}**, R**_{**5**} et **R**_{**6**} ensemble, deux à deux, forment, avec l'atome d'azote auxquels ils sont rattachés, un cycle saturé carboné à 4, 5, 6 ou 7 chaînons pouvant contenir un ou plusieurs hétéroatomes, le cycle pouvant être substitué par un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C,-C₆)silanealkyle en C₁-C₆, un radical carbamoyle, un radical carboxyle, un radical alkyl(C₁-C₆)carbonyle, un radical amino, un radical amino mono- ou di-substitué par un radical alkyl(C₁-C₆), alkyl(C₁-C₆)carbonyle, carbamoyle ou alkyl(C₁-C₆)sulfonyle;
- **R**_{**7**} représente un radical alkyle en C₁-C₆; un radical monohydroxyalkyle en C₁-C₆; un radical polyhydroxyalkyle en C₂-C₆ ; un radical aryle ; un radical benzyle; un radical aminoalkyle en C₁-C₆; un radical aminoalkyle en C₁-C₆ dont l'amine est mono- ou di-subsitué par un radical alkyl(C₁-C₆), alkyl(C₁-C₆)carbonyle, carbamoyle ou alkyl(C₁₋C₆)sulfonyle ; un radical carboxyalkyle en C₁-C₆ ; un radical carbamoylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical N-alkyl(C₁₋C₆)carbamoylalkyle en C₁-C₆ ; un radical N-alkyl(C₁-C₆)sulfonamidoalkyle en C₁-C₆ ;
   - x est 0 ou 1,
      - lorsque x = 0, alors le bras de liaison est rattaché à l'atome d'azote portant les radicaux R₅, R₆ et R₈
      - lorsque x = 1, alors deux des radicaux R₅, R₆ et R₈ forment conjointement avec l'atome d'azote auquel ils sont rattachés un cycle saturé à 4, 5, 6 ou 7 chaînons et D est lié à un atome de carbone du cycle saturé;
   - Y⁻ est un contre-ion.

Selon ce mode de réalisation, x est de préférence égal à 0, et R₅ , R₆ et R₈ sont séparément choisis parmi un radical alkyle en C₁-C₆, un radical monohydroxyalkyle en C₁-C₄, un radical polyhydroxyalkyle en C₂-C₄, un radical alcoxy(C₁-C₆)alkyle en C₁-C₄, un radical carbamoylalkyle en C₁-C₆, un radical trialkyl(C₁-C₆)silanealkyle en C₁-C₆ , ou R₈ avec R₅ forment ensemble un cycle azétidine, pyrrolidine, pipéridine, pipérazine, morpholine, R₆ étant choisi dans ce cas parmi un radical alkyle en C₁-C₆; un radical monohydroxyalkyle en C₁-C₆ ; un radical polyhydroxyalkyle en C₂-C₆; un radical aminoalkyle en C₁-C₆, un radical aminoalkyle mono- ou di-substitué par un radical alkyl(C₁-C₆), alkyl(C₁-C₆)carbonyle, carbamoyle ou alkyl(C₁-C₆)sulfonyle ; un radical carbamoylalkyle en C₁-C₆ ; un radical trialkyl(C₁₋C₆)silanealkyle en C₁-C₆ ; un radical alkyl(C₁-C₆)carboxyalkyle en C₁-C₆ ; un radical alkyl(C₁-C₆)carbonylalkyle en C₁-C₆ ; un radical N-alkyl(C₁-C₆)carbamoylalkyle en C₁-C₆.

Selon un autre mode de réalisation, x est égal à 1, R₇ est choisi parmi un radical alkyle en C₁-C₆ ; un radical monohydroxyalkyle en C₁-C₆ ; un radical polyhydroxyalkyle en C₂-C₆ ; un radical aminoalkyle en C₁-C₆, un radical aminoalkyle en C₁-C₆ dont l'amine est mono ou disubstituée par un radical alkyl(C₁-C₆), alkyl(C₁₋C₆)carbonyle, carbamoyle ou alkyl(C₁-C₆)sulfonyle ; un radical carbamylalkyle en C₁-C₆ ; un radical trialkyl(C₁-C₆)silanealkyle en C₁-C₆ ; un radical alkyl(C₁-C₆)carboxyalkyle en C₁-C₆ ; un alkyl(C₁-C₆)carbonylalkyle en C₁-C₆ un radical N-alkyl(C₁-C₆)carbamylalkyle en C₁-C₆ ; R₈ avec R₅ ensemble forment un cycle azétidine pyrrolidine, pipéridine, pipérazine, morpholine, R₆ étant choisi dans ce cas parmi un radical alkyle en C₁-C₆ ; un radical monohydroxyalkyle en C₁-C₆ un radical polyhydroxyalkyle en C₂-C₆; un radical aminoalkyle en C₁-C₆, un radical aminoalkyle en C₁-C₆ dont l'amine est mono ou disubstituée par un radical alkyl(C₁-C₆), alkyl(C₁-C₆)carbonyle, carbamoyle ou alkyl(C,-C₆)sulfonyle; un radical carbamoylalkyle en C₁-C₆; un radical trialkyl(C₁-C₆)silanealkyle en C₁-C₆ ; un radical alkyl(C₁-C₆)carboxyalkyle en C₁-C₆ ; un radical alkyl(C₁₋C₆)carbonylalkyle en C₁-C₆ ; un radical N-alkyl(C₁-C₆)carbamoylalkyle en C₁-C₆.

A titre d'exemple préféré selon ce mode de réalisation, R₂ est un radical trialkylammoniumalkyle, l'alkyle reliant R2 au cycle pouvant être substitué par un ou plusieurs groupements hydroxy.

De préférence, D est une liaison covalente ou une chaîne alkylène pouvant être substituée et pouvant contenir un groupe carbonyle.

Selon un second mode de réalisation, R₂ représente un radical onium Z correspondant à la formule (III) 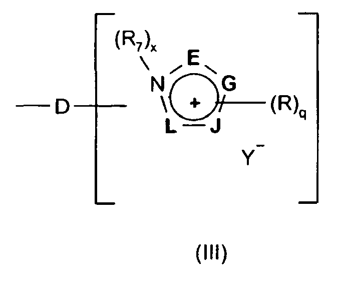 dans laquelle
- D est une liaison covalente ou une chaîne alkylène en C₁-C₁₄, linéaire ou ramifiée pouvant contenir un ou plusieurs hétéroatomes choisis parmi l'oxygène, le soufre ou l'azote, et pouvant être substituée par un ou plusieurs radicaux hydroxyle ou amino, et pouvant comprendre un ou plusieurs carbonyles;
- les sommets E, G, J, L, identiques ou différents, représentent un atome de carbone, d'oxygène, de soufre ou d'azote pour former un cycle pyrrolique, pyrazolique, imidazolique, triazolique, oxazolique, isooxazolique, thiazolique, isothiazolique,
- q est un nombre entier compris entre 1 et 4;
- **R,** identiques ou différents, représentent un atome d'hydrogène, un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical carbamoyle, un radical carboxyle, un radical alkylcarbonyle en C₁-C₆, un radical amino, un radical amino mono ou di substituée par un radical alkyl(C₁-C₆), alkyl(C₁-C₆)carbonyle, carbamoyle ou alkyl(C₁-C₆)sulfonyle, un radical monohydroxyalkyle en C₁-C₆, un radical polyhydroxyalkyle en C₂-C₆ ou un radical ammonium quaternaire ;
- **R**_{**7**} représente un radical alkyle en C₁-C₆ ; un radical monohydroxyalkyle en C₁₋C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est substitué par un radical alkyl(C₁-C₆), alkyl(C₁-C₆)carbonyle, carbamoyle ou alkyl(C₁-C₆)sulfonyle ; un radical carboxyalkyle en C₁-C₆ ; un radical carbamoylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁₋C₆)carboxyalkyle en C₁-C₆ ; un radical N-alkyl(C₁-C₆)carbamoylalkyle en C₁-C₆ ; un radical N-alkyl(C₁-C₆)sulfonamidoalkyle en C₁-C₆ ; un radical ammonium quaternaire ;
- x est 0 ou 1
   - lorsque x = 0, le bras de liaison D est rattaché à l'atome d'azote,
   - lorsque x = 1, le bras de liaison D est rattaché à l'un des sommets E, G, J ou L,
- Y⁻ est un contre-ion.

Selon ce mode de réalisation, les sommets E, G, J et L forment de préférence un cycle pyrrolique, imidazolique, pyrazolique, oxazolique, thiazolique et triazolique. De préférence, les sommets E, G, J et L forment un cycle imidazolique.

Lorsque x est égal à 0, D est de préférence une liaison covalente ou une chaîne alkylène pouvant être substituée et/ou contenir une fonction carbonyle.

Selon un troisième mode de réalisation, R₂ représente un radical onium Z correspondant à la formule (IV) 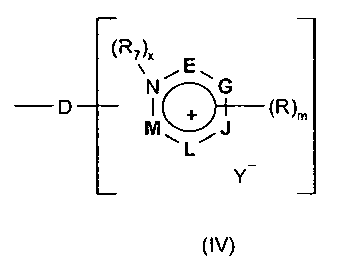 dans laquelle :
- D est une liaison covalente ou une chaîne alkylène en C₁-C₁₄, linéaire ou ramifiée pouvant contenir un ou plusieurs hétéroatomes choisis parmi un atome d'oxygène, de soufre ou d'azote, et pouvant être substituée par un ou plusieurs radicaux hydroxyle ou amino, et pouvant comprendre un ou plusieurs carbonyles ;
- les sommets E, G, J, L et M identiques ou différents, représentent un atome de carbone, d'oxygène, de soufre ou d'azote et forment un cycle choisi parmi les cycles pyridiniques, pyrimidiniques, pyraziniques, triaziniques et pyridaziniques,
- **m** est un nombre entier compris entre 1 et 5;
- **R,** identiques ou différents, représentent un atome d'hydrogène, un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical carbamoyle, un radical carboxyle, un radical alkylcarbonyle en C₁-C₆, un radical amino, un radical amino substitué par un radical alkyl(C₁-C₆), alkyl(C₁₋C₆)carbonyle, carbamoyle ou alkyl(C₁-C₆)sulfonyle, un radical monohydroxyalkyle en C₁-C₆ ou un radical polyhydroxyalkyle en C₂-C₆ ; un radical amonium quaternaire ,
- **R**_{**7**} représente un radical alkyle en C₁-C₆; un radical monohydroxyalkyle en C₁₋C₆ ; un radical polyhydroxyalkyle en C₂-C₆ ; un radical aryle ; un radical benzyle; un radical aminoalkyle en C₁-C₆, un radical aminoalkyle en C₁-C₆ dont l'amine est mono- ou di- substituée par un radical alkyl(C₁-C₆), alkyl(C₁-C₆)carbonyle, carbamoyle ou alkyl(C₁-C₆)sulfonyle; un radical carboxyalkyle en C₁-C₆; un radical carbamoylalkyle en C₁-C₆; un radical trifluoroalkyle en C₁-C₆; un radical trialkyl(C₁-C₆)silanealkyle en C1-C6; un radical sulfonamidoalkyle en C₁-C₆; un radical alkyl(C₁-C₆)carboxyalkyle en C₁-C₆; un radical alkyl(C₁-C₆)sulfinylalkyle en C₁-C₆; un radical alkyl(C₁-C₆)sulfonylalkyle en C₁-C₆; un radical alkyl(C₁₋C₆)carboxyalkyle en C₁-C₆; un radical N-alkyl(C₁-C₆)carbamoylalkyle en C₁-C₆ ; un radical N-alkyl(C₁-C₆)sulfonamidoalkyle en C₁-C₆; un radical ammonium quaternaire ;
- **x** est 0 ou 1
   - lorsque x = 0, le bras de liaison D est rattaché à l'atome d'azote,
   - lorsque x = 1, le bras de liaison D est rattaché à l'un des sommets E, G, J, L ou M,
**27.** Y⁻ représente un contre ion.

Dans ce mode opératoire, les sommets E,G,J,L et M avec l'azote du cycle forment de préférence un cycle choisi parmi les cycles pyridiniques, pyrimidiniques.

De préférence, lorsque x est égal à 0 alors **R** est choisi parmi un atome d'hydrogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical carbamoyle, un radical alkylcarbonyle en C₁-C₆, un radical amino, un radical amino mono- ou di-substitué par un radical alkyl(C₁-C₆), alkyl(C₁-C₆)carbonyle, carbamoyle ou alkyl(C₁₋C₆)sulfonyle ; un radical monohydroxyalkyle en C₁-C₆ ou un radical polyhydroxyalkyle en C₂-C₆.

De préférence, lorsque x est égal à 1, **R**_{**7**} est choisi parmi un radical alkyle en C₁-C₆ ; un radical monohydroxyalkyle en C₁-C₆ ; un radical polyhydroxyalkyle en C₂-C₆ ; un radical aminoalkyle en C₁-C₆, un radical aminoalkyle en C₁-C₆ dont l'amine est mono- ou di- substituée par un radical alkyl(C₁-C₆), alkyl(C₁-C₆)carbonyle, un radical carbamoyle ou un radical alkyl(C₁-C₆)sulfonyle ; un radical carbamoylalkyle en C₁-C₆ ; un radical trialkyl(C₁-C₆)silanealkyle en C₁-C₆ ; un radical alkyl(C₁₋C₆)carbonylalkyle en C₁-C₆ ; un radical N-alkyl(C₁-C₆)carbamylalkyle en C₁-C₆ ; **R** est choisi parmi un atome d'hydrogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical carbamoyle, un radical alkylcarbonyle en C₁-C₆, un radical amino, un radical amino mono- ou di-substitué par un radical alkyl(C₁-C₆), alkyl(C₁-C₆)carbonyle, carbamoyle ou alkyl(C₁₋C₆)sulfonyle.

De préférence, **R** est choisi parmi un atome d'hydrogène ou des radicaux alkyles pouvant être substitués et R₇ est un radical alkyle pouvant être substitué.

Selon un mode de réalisation particulier, **R** est choisi parmi l'hydrogène ; un radical alkyle ; un radical alkyle substitué par un ou plusieurs hydroxy ; un radical alkyle substitué par un ou plusieurs amino ; un radical carboxyle, un radical carbamoyle. A titre d'exemple, on peut citer pour **R** l'hydrogène ; le radical hydroxyle ; le radical méthyle ; le radical amino ; le radical hydroxyméthyle ; le radical aminométhyle, de préférence l'hydrogène.

Dans la formule (I) ci-dessus, b est de préférence égal à 0 ou **R**_{**4**} est de préférence choisi parmi un radical alkyle ; un radical alkyle substitué par un ou plusieurs hydroxy ; un radical alkyle substitué par un ou plusieurs amino ; un radical carboxyle ; un radical carbamoyle, de préférence l'hydrogène.

Dans la formule (I) ci-dessus, R₃ est de préférence choisi parmi l'hydrogène ; le radical hydroxyle ; le radical amino ; un radical alkyle; un radical alkyle substitué par un ou plusieurs hydroxy ; un radical alkyle substitué par un ou plusieurs amino ; un radical carboxyle ; un radical carbamoyle, de préférence l'hydrogène.

Le carbone substitué par R₃ ou par R₄, peut être de configuration (R) et/ou (S).

Les contre ions Y⁻ sont par exemple choisis parmi un atome d'halogène, un hydroxyde, un hydrogènesulfate, un acétate, un tartrate ou un alkyl(C₁-C₆)sulfate tel que par exemple un méthylsulfate ou un éthylsulfate.

A titre d'exemples de dérivés de formule (1), on peut citer :

| formule | Nomenclature | formule | nomenclature |
|---|---|---|---|
| 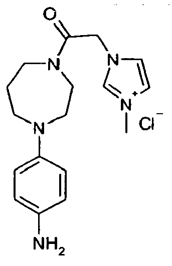 | 3-{2-[4-(4-Amino-phényl)-[1,4]diazépan-1-yl]-2-oxo-éthyl}-1-méthyl-3H-imidazol-1-um ; chlorure | 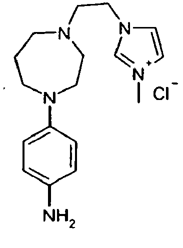 | 3-{2-[4-(4-Amino-phényl)-[1,4]diazépan-1-yl]-éthyl}-1-méthyl-3H-imidazol-1-ium; chlorure |
| 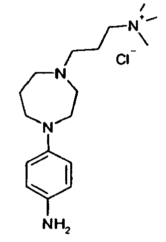 | {3-[4-(4-Amino-phényl)-[1,4]diazépan-1-yl]-propyl}-triméthyl-ammonium; chlorure | 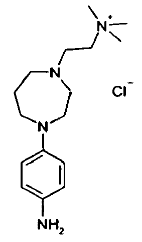 | {2-[4-(4-Amino-phényl)-[1,4]diazépan-1-yl]-éthyl}-triméthyl-ammonium; chlorure |
| 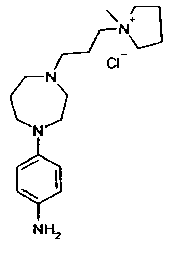 | 1-{3-[4-(4-Amino-phényl)-[1,4]diazépan-1-yl]-propyl}-1-méthyl-pyrrolidinium; chlorure | 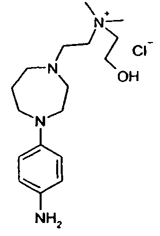 | {2-[4-(4-Amino-phényl)-[1,4]diazépan-1-yl]-éthyl}-(2-hydroxy-éthyl)-diméthyl-ammonium ; chlorure |
| 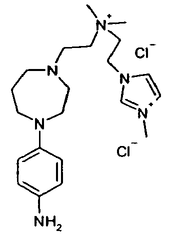 | {3-[4-(4-Amino-phényl)-[1,4]diazépan-1-yl]-éthyl}-diméthyl(éthyl-2-méthyl-3H-imidazol-1-ium chlorure) -ammonium; chlorure | 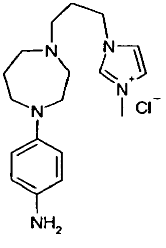 | 3-{2-[4-(4-Amino-phényl)-[1,4]diazépan-1-yl]-propyl}-1-méthyl-3H-imidazol-1-ium; chlorure |
| 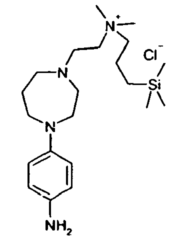 | {2-[4-(4-Amino-phényl)-[1,4]diazépan-1-yl]-éthyl}-diméthyl-(3-triméthylsilanyl-propyl)-ammonium ; chlorure | 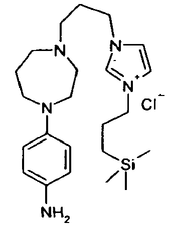 | 3-{2-[4-(4-Amino-phényl)-[1,4]diazépan-1-yl]-éthyl}-1-(3-triméthylsilanyl-propyl)-3H-imidazol-1-ium ; chlorure |
| 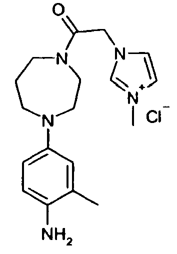 | 3-{2-[4-(4-Amino-3-méthyl-phényl)-[1,4]diazépan-1-yl]-2-oxo-éthyl}-1-méthyl-3H-imidazol-1-um ; chlorure | 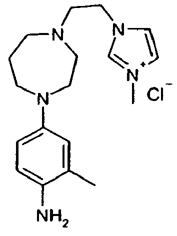 | 3-{2-[4-(4-Amino-3-méthyl-phényl)-[1,4]diazépan-1-yl]-éthyl}-1-méthyl-3H-imidazol-1-um ; chlorure |
| 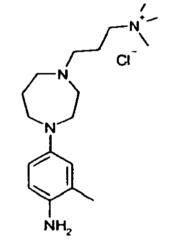 | {3-[4-(4-Amino-3-méthyl-phényl)-[1,4]diazépan-1-yl]-propyl}-triméthyl-ammonium; chlorure | 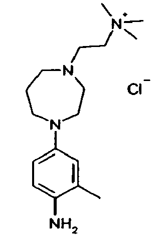 | {2-[4-(4-Amino-3-méthyl-phényl)-[1,4]diazépan-1-yl]-éthyl}-triméthyl-ammonium; chlorure |
| 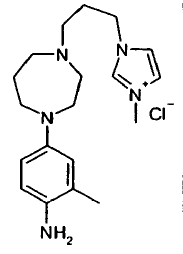 | 3-{2-[4-(4-Amino-3-méthyl-phényl)-[1,4]diazépan-1-yl]-propyl}-1-méthyl-3H-imidazol-1-um ; chlorure | 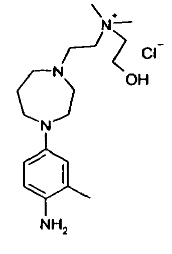 | {2-[4-(4-Amino-3-méthyl-phényl)-[1,4]diazépan-1-yl]-éthyl}-(2-hydroxy-éthyl)-diméthyl-ammonium ; chlorure |
| 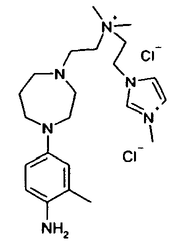 | {3-[4-(4-Amino-3-méthyl-phényl)-[1,4]diazépan-1-yl]-éthyl}-diméthyl(éthyl-2--méthyl-3H-imidazol-1-ium chlorure) -ammonium; chlorure | 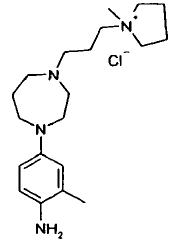 | 1-{3-[4-(4-Amino-3-méthyl-phényl)-[1,4]diazépan-1-yl]-propyl}-1-méthyl-pyrrolidinium ; chlorure |
| 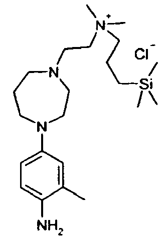 | {2-[4-(4-Amino-3-méthyl-phényl)-[1,4]diazépan-1-yl]-éthyl}-diméthyl-(3-triméthylsilanyl-propyl)-ammonium ; chlorure | 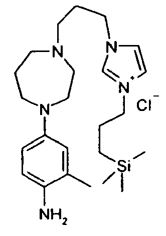 | 3-{2-[4-(4-Amino-3-méthyl-phényl)-[1,4]diazépan-1-yl]-éthyl}-1-(3-triméthylsilanyl-propyl)-3H-imidazol-1-ium ; chlorure |
| 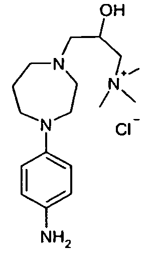 | {3-[4-(4-Amino-phényl)-[1,4]diazepan-1-yl]-2-hydroxy-propyl}-triméthyl-ammonium; chlorure | 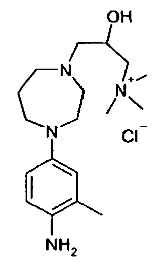 | {3-[4-(4-Amino-3-méthyl-phényl)-[1,4]diazépan-1-yl]-2-hydroxy-propyl}-triméthyl-ammonium; chlorure |
| 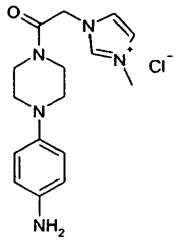 | 3-{2-[4-(4-Amino-phényl)-piperazin-1-yl]-2-oxo-éthyl}-1-méthyl-3H-imidazol-1-ium; chlorure | 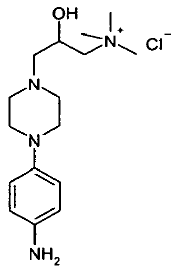 | {3-[4-(4-Amino-phényl)-piperazin-1-yl]-2-hydroxy-propyl}-triméthyl-ammonium; chlorure |
| 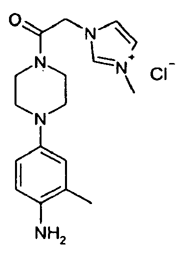 | 3-{2-[4-(4-Amino-3-méthyl-phényl)-pipérazin-1-yl]-2-oxo-éthyl}-1-méthyl-3H-imidazol-1-ium; chlorure | 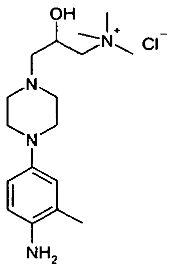 | 3-[4-(4-Amino-3-méthyl-phényl)-pipérazin-1-yl]-2-hydroxy-propyl}-triméthyl-ammonium; chlorure |
| 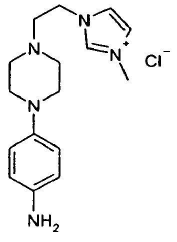 | 3-{2-[4-(4-Amino-phényl)-pipérazin-1-yl]-éthyl}-1-méthyl-3H-imidazol-1-ium; chlorure | 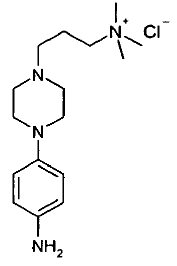 | {3-[4-(4-Amino-phényl)-piperazin-1-yl]-propyl}-triméthyl-ammonium; chlorure |
| 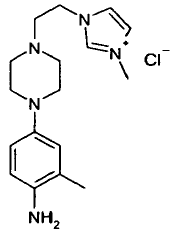 | 3-{2-[4-(4-Amino-3-méthyl-phényl)-pipérazin-1-yl]-éthyl}-1-méthyl-3H-imidazol-1-ium; chlorure | 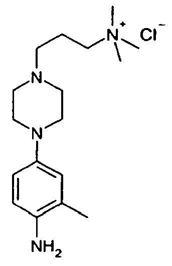 | {3-[4-(4-Amino-3-méthyl-phényl)-pipérazin-1-yl]-propyl}-triméthyl-ammonium; chlorure |
| 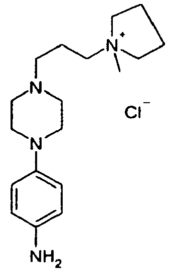 | 1-{3-[4-(4-Amino-phényl)-piperazin-1-yl]-propyl}-1-méthyl-pyrrolidinium; chlorure | | |
| 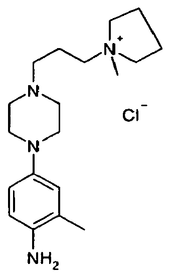 | 1 -{3-[4-(4-Amino-3-méthyl-phényl)-piperazin-1-yl]-propyl}-1-méthyl-pyrrolidinium; chlorure | | |

De préférence, les dérivés de formule (1) sont choisis parmi :
- 3-{2-[4-(4-Amino-phényl)-[1,4]diazépan-1-yl]-2-oxo-éthyl}-1-méthyl-3H-imidazol-1-um ; chlorure
- 3-{2-[4-(4-Amino-phényl)-[1,4]diazépan-1-yl]-éthyl}-1-méthyl-3H-imidazol-1-ium; chlorure
- {3-[4-(4-Amino-phényl)-[1,4]diazépan-1-yl]-propyl}-triméthyl-ammonium; chlorure
- {2-[4-(4-Amino-phényl)-[1,4]diazépan-1-yl]-éthyl}-triméthyl-ammonium; chlorure
- 1-{3-[4-(4-Amino-phényl)-[1,4]diazépan-1-yl]-propyl}-1-méthyl-pyrrolidinium; chlorure
- {2-[4-(4-Amino-phényl)-[1,4]diazépan-1-yl]-éthyl}-(2-hydroxy-éthyl)-diméthyl-ammonium ; chlorure
- 3-{2-[4-(4-Amino-phényl)-[1,4]diazépan-1-yl]-propyl}-1-méthyl-3H-imidazol-1-ium; chlorure
- {3-[4-(4-Amino-phényl)-[1,4]diazépan-1-yl]-2-hydroxy-propyl}-triméthyl-ammonium; chlorure
- 3-{2-[4-(4-Amino-phényl)-piperazin-1-yl]-2-oxo-éthyl}-1-méthyl-3H-imidazol-1-ium; chlorure
- {3-[4-(4-Amino-phényl)-piperazin-1-yl]-2-hydroxy-propyl}-triméthyl-ammonium; chlorure
- 3-{2-[4-(4-Amino-phényl)-pipérazin-1-yl]-éthyl}-1-méthyl-3H-imidazol-1-ium; chlorure
- {3-[4-(4-Amino-phényl)-piperazin-1-yl]-propyl}-triméthyl-ammonium; chlorure
- 1-{3-[4-(4-Amino-phényl)-piperazin-1-yl]-propyl}-1-méthyl-pyrrolidinium; chlorure

Les dérivés de formule (1) peuvent être obtenus par exemple à partir des synthèses décrites dans la demande de brevet FR 2 828 488.

Les dérivés cationiques peuvent être obtenus par la voie de synthèse suivante :

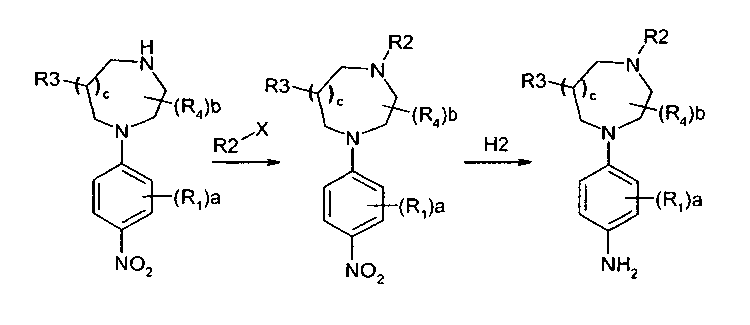

La présente invention a aussi pour objet, les dérivés nitro utiles dans la synthèse des dérivés de formule (I). Ces dérivés nitro correspondent à la formule (I') suivante 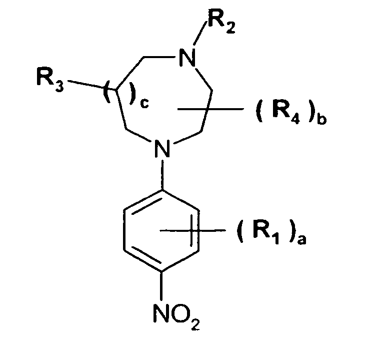 dans laquelle R₁, R₂, R₃, R₄, a, b et c sont tels que définis précédemment.

La composition tinctoriale de la présente invention comprend, dans un milieu approprié pour la teinture des fibres kératiniques, en particulier les cheveux humains, à titre de base d'oxydation un dérivé de formule (I) tel que défini précédemment.

La ou les bases d'oxydation de l'invention sont en général présentent chacune en quantité comprise entre 0,001 à 10 % en poids environ du poids total de la composition tinctoriale, de préférence entre 0,005 et 6 %.

La composition tinctoriale de l'invention peut contenir un ou plusieurs coupleurs conventionnellement utilisés pour la teinture de fibres kératiniques. Parmi ces coupleurs, on peut notamment citer les métaphénylènediamines, les méta-aminophénols, les métadiphénols, les coupleurs naphtaléniques, les coupleurs hétérocycliques et leur sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

La composition de la présente invention peut en outre comprendre une ou plusieurs bases d'oxydation additionnelles classiquement utilisées en teinture d'oxydation autres que celles décrites précédemment. A titre d'exemple, ces bases d'oxydation additionnelles sont choisies parmi les paraphénylènediamines autres que celles décrites précédemment, les bis-phénylalkylènediamines, les para-aminophénols, les bis-paraaminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétcarbamoyle 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1 ,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation présentes dans la composition de l'invention sont en général présentent chacune en quantité comprise entre 0,001 à 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 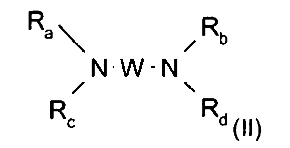 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, et on révèle la couleur à l'aide d'un agent oxydant. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale de la présente invention définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale comprenant au moins une base d'oxydation de formule (I) avec un agent oxydant, et l'application du mélange obtenu sur les fibres kératiniques pendant un temps suffisant pour développer la coloration désirée.

Les exemples qui suivent servent à illustrer l'invention sans toutefois présenter un caractère limitatif.

### EXEMPLES

### Exemple 1 : Préparation du 3-{2-[4-(4-Amino-phényl)-[1,4]diazépan-1-yl]-2-oxo-éthyl}-1-méthyl-3H-imidazol-1-um ; chlorure ; dichlorhydrate ( 4 )

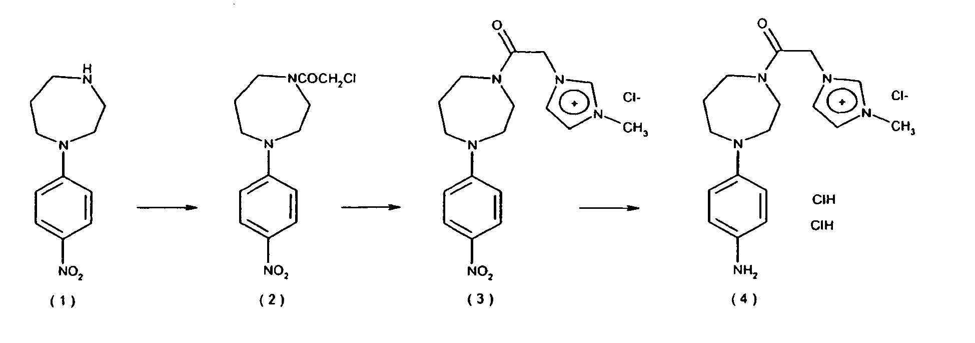

### 1^{ère} étape : synthèse du 2-chloro-1-[4-(4-nitro-phényl)-[1,4]diazépan-1-yl]-éthanone ( 2)

Dans un réacteur, sont mélangés 22,1 g (0,1 mole) de 1-(4-nitro-phényl)-[1,4]diazépane, 8,3g (0,06mole) de carbonate de potassium et 120 ml de diméthylformamide . A cette suspension agitée, sont ajoutés 8,3 ml (0, 11 mole) de chlorure de chloracétyle en maintenant la température entre 15 et 25°C. Après une agitation pendant une dizaine d'heures à température ambiante, 400g d'eau glacée légèrement chlorhydrique sont ajoutés au mélange : une gomme jaune cristallise. Après essorage, lavage à l'eau et séchage sous vide sur anhydride phosphorique, 22,4g de cristaux jaunes sont obtenus (rendement = 75%).

RMN¹H (400 MHz, DMSO D6) :1,83 (m), 1,9 (m), 3,37 (t), 3,47 (t ), 3,67 (m), 3,85 (t ), 4,28 (s), 4,36 (s), 6,9 (m) 8,04 (m)

### 2^{éme} étape : synthèse du 3-méthyl-1-{2-[4-(4-nitro-phényl)-[1,4]diazépan-1-yl]-2-oxo-éthyl}-3H-imidazol-1-ium; chlorure (3)

Sous agitation, un mélange de 19,95g (0,067mole) de 2-chloro-1-[4-(4-nitro-phényl)-[1,4]diazépan-1-yl]-ethanone obtenu ci-dessus à l'étape précédente et 11,5 g (0,14mole) de 1-méthyl-1H-imidazole dans 40 ml d'isobutanol sont chauffés au reflux pendant 1 heure 30. Après cristallisation à froid, un précipité de cristaux jaunes est obtenu. Ce précipité est ensuite lavé à l'isobutanol puis à l'éther éthylique et séché à 40°C sous vide et sur anhydride phosphorique.
Après recristallisation de 65ml d'éthanol au reflux, 14,0 g d'un composé cristallisé jaune sont obtenus (rendement = 55%).

RMN¹H (400 MHz, DMSO, D6) :1,85-2 (2m, 2H), 3,37-3,5 (2m, 2H), 3,72-3,89-3,95 (2m + 2s, 9H), 5,385-5,389 (2s, 2H), 6,93 (2m, 2H), 7,61 (2dd ,1H), 7,68 (2dd, 1 H), 8,05 (2m, 2H), 9,08-9,1 (2dd, 1H)

### 3^{ème} étape : synthèse du 3-{2-[4-(4-Amino-phényl)-[1,4]diazépan-1-yl]-2-oxo-éthyl}-1-méthyl-3H-imidazol-1-um, chlorure , dichlorhydrate ( 4 )

Dans un hydrogènateur d'un litre sont placés 13,1 g (0,0345 mole) de 3-méthyl-1-{2-[4-(4-nitro-phényl)-[1,4]diazépan-1-yl]-2-oxo-éthyl}-3H-imidazol-1-ium, chlorure, obtenu à l'étape précédente, 3 g de palladium sur charbon (contenant 50% d'eau), 400ml d'éthanol à 96 et 150 ml d'eau.
La réduction est effectuée en deux heures sous une pression d'hydrogène d'environ cinq bars et à une température de 70°C. Après filtration du catalyseur sous azote, de l'acide chlorhydrique aqueux est coulé sur le mélange.
Après évaporation du filtrat sous pression réduite, recristallisation du mélange au reflux d'éthanol, acide chlorhydrique et eau et séchage à 40°C sous vide et sur potasse, 15,9g (rendement = 75%) de cristaux blancs sont obtenus.

Analyse élémentaire calculée pour C₁₇H₂₆N₅OCl₃ + H₂O + CH₃CH₂OH est :

| | C% | H% | N% | O% | Cl% |
|---|---|---|---|---|---|
| Calculé : | 48,87 | 7,04 | 14,38 | 9,86 | 21,85 |
| Trouvé : | 46,82 | 6,89 | 14,42 | 9,41 | 22,39 |

Les spectres RMN ¹³C et RMN ¹H sont en accord avec la structure attendue.

RMN¹H (400 MHz, D₂O) : 2,21-2,47 (2m, 2H), 3,78-3,89 (m, 4H), 3,97-4,09 (m + s, 7H), 5,27-5,42 (2 H), 7,33-7,72 (m, 6H), 8,71-8,8 (2s, 1 H)

RMN ¹³C (D₂O) : 23,25 ; 24,32 ; 35,89 ; 41,77 ; 43,44 ; 44,96 ; 45,42 ; 50,17 ; 53,98 ; 55,94 ; 54,59 ; 56,5 ; 118,25 ; 120,69 ; 123,1 ; 123,27 ; 123,55 ; 123,68 ; 124,9 ; 124,99 ; 125,53 ; 128,71 ; 137 ; 143,6 ; 144,8 ; 166,16 ; 166,74

Le spectre de masse montre que le cation attendu, C₁₇H₂₄N₅O+, est principalement détecté à m/z=314 en ES+.

### Exemple 2 : 2-hydroxy-N,N,N-triméthyl-3-[4-(4-aminophényl)-1,4-diazépan-1-yl]propan-1-aminium acétate (3)

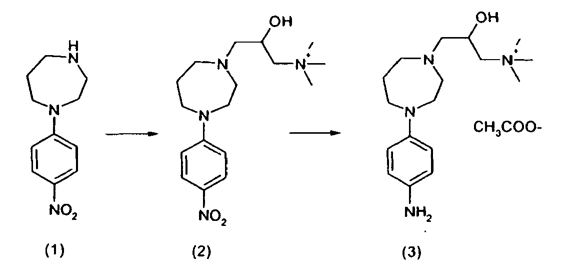

### Synthèse du 2-hydroxy-N,N,N-triméthyl-3-[4-(4-nitrophényl)-1,4-diazépan-1-yl]propan-1-aminium acetate (2)

Dans un tricol, on introduit 1.1 g de 1-(4-nitrophényl)-1,4-diazépane (0,005 mole), 3 ml de DMF et 0.76g (0,00448mole)de glycidyltriméthylamonium. Le mélange est chauffé à 105° pendant 20 heures. Le mélange réactionnel est ensuite versé dans 50ml d'acétate d'éthyle. Après trituration de la gomme et élimination de l'acétate d'éthyle , on reprend le produit dans l'eau, extrait au butanol et concentre la phase aqueuse. Un échantillon est purifié par HPLC préparative.

RMN ¹H (DMSO d₆, 200 MHz, ppm) conforme au produit attendu : 8,03 (d, 2H) ; 6,84 (d, 2H) ; 4,25(m , 1 H) ; 3,23-3,67 (m, 8H) ; 3,095 (s, 9H) ; 2,823 (m, 2H) ; 2,5-2,615 (m, 2H) ; 1,83 (m, 2H) ; 1,69 (s, 3H)

Masse ESI+ : m /z=337[M+]

### Synthèse du 2-hydroxy-N,N,N-triméthyl-3-[4-(4-aminophényl)-1,4-diazépan-1-yl]propan-1-aminium acetate (3)

Après réduction au zinc/ acide acétique, on obtient le 2-hydroxy-N,N,N-triméthyl-3-[4-(4-aminophényl)-1,4-diazépan-1-yl]propan-1-aminium acétate

Masse ESI+ : m /z=307[M+]

### EXEMPLES de teinture

### EXEMPLES 1 A 7: Teinture en milieu alcalin à partir du 3-{2-[4-(4-Amino-phényl)-[1,4]diazépan-1-yl]-2-oxo-éthyl}-1-méthyl-3H-imidazol-1-um ; chlorure ; dichlorhydrate

Les compositions tinctoriales suivantes ont été préparées :

| **Exemples** | **1** | **2** | **3** | **4** | **5** | **6** | **7** |
|---|---|---|---|---|---|---|---|
| 3-{2-[4-(4-Amino-phényl)-[1,4]diazépan-1-yl]-2-oxo-éthyl}- 1-méthyl-3H-imidazol-1-um ; chlorure ; dichlorhydrate (base) | 10⁻³ mole | 10⁻³ mole | 10⁻³ mole | - | - | - | - |
| 2-hydroxy-N,N,N-triméthyl-3-[4-(4-aminophényl)-1,4-diazépan-1- yl]propan-1-aminium acetate (Base) | - | - | - | 10⁻³ mole | 10⁻³ mole | 10⁻³ mole | 10⁻³ mole |
| 2-(2,4-Diamino-phénoxy)-éthanol, dichlorhydrate (coupleur) | 10⁻³ mole | - | - | 10⁻³ mole | - | - | - |
| 3-Amino-2-chloro-6-méthyl-phénol, chlorhydrate (coupleur) | - | 10⁻³ mole | - | - | 10⁻³ mole | - | - |
| 3,6-Diméthyl-1H-pyrazolo[5,1-c] [1,2,4]triazole (coupleur) | - | - | 10⁻³ mole | - | - | 10⁻³ mole | - |
| 2-méthyl-5-aminophénol (coupleur) | | | | | | | 10⁻³ mole |
| Support de teinture (1) | (*) | (*) | (*) | (*) | (*) | (*) | (*) |
| Eau déminéralisée q.s.p. | 100 g | 100 g | 100 g | 100 g | 100 g | 100 g | 100 g |

| | | | | | | | |
|---|---|---|---|---|---|---|---|
| (*) Support de teinture (1) pH 9,5 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g NH₄Cl 4,32 g Ammoniaque à 20% de NH3 2,94 g | | | | | | | |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 min de pause, les mèches sont rinçées, lavées avec un shampooing standard, rinçées à nouveau puis séchées.
Les résultats de teinture suivants ont été obtenus.

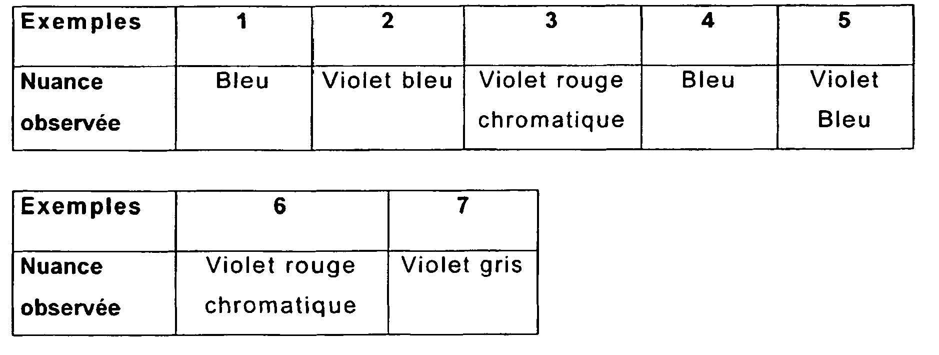

### EXEMPLES 8 A 16 : Teinture en milieu acide à partir du 3-{2-[4-(4-Amino-phényl)-[1,4]diazépan-1-yl]-2-oxo-éthyl}-1-méthyl-3H-imidazol-1-um ; chlorure ; dichlorhydrate

Les compositions tinctoriales suivantes ont été préparées :

| **Exemples** | **8** | **9** | **10** | **11** |
|---|---|---|---|---|
| 3-{2-[4-(4-Amino-phényl)-[1,4]diazépan-1-yl]-2-oxo-éthyl}-1-méthyl-3H-imidazol-1-um ; chlorure ; dichlorhydrate (base) | 10⁻³ mole | 10⁻³ mole | 10⁻³ mole | 10⁻³ mole |
| 2-(2,4-Diamino-phénoxy)-éthanol, dichlorhydrate (coupleur) | 10⁻³ mole | | | |
| 3-Amino-2-chloro-6-méthyl-phénol, chlorhydrate (coupleur) | | 10⁻³ mole | | |
| 2-méthyl-5-aminophénol (coupleur) | | | 10⁻³ mole | |
| 2-Amino-pyridin-3-ol (coupleur) | | | | 10⁻³ mole |
| Support de teinture (2) | (*) | (*) | (*) | (*) |
| Eau déminéralisée q.s.p. | 100 g | 100 g | 100 g | 100 g |

| **Exemples** | **12** | **13** | **14** | **15** | **16** |
|---|---|---|---|---|---|
| 2-hydroxy-N, N, N-triméthyl-3-[4-(4-aminophényl)-1,4-diazépan-1-yl]propan-1-aminium acetate (Base) | 10⁻³ mole | 10⁻³ mole | 10⁻³ mole | 10⁻³ mole | 10⁻³ mole |
| 2-(2,4-Diamino-phénoxy)-éthanol, dichlorhydrate (coupleur) | 10⁻³ mole | - | - | - | - |
| 3-Amino-2-chloro-6-méthyl-phénol, chlorhydrate (coupleur) | - | 10⁻³ mole | - | - | - |
| 2-méthyl-5-aminophénol (coupleur) | - | - | 10⁻³ mole - | | - |
| 2-Amino-pyridin-3-ol (coupleur) | - | - | - | 10⁻³ mole | |
| 6-Hydroxy-1-H-indole (coupleur) | - | - | - | - | 10⁻³ mole |
| Support de teinture (2) | (*) | (*) | (*) | (*) | (*) |
| Eau déminéralisée q.s.p. | 100 g | 100 g | 100 g | 100 g | 100 g |

| | | | | | |
|---|---|---|---|---|---|
| (*) Support de teinture (2) pH 7 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g Na₂HPO₄ 0,28 g KH₂PO₄ 0,46 g | | | | | |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 min de pose, les mèches sont rinçées, lavées avec un shampooing standard, rinçées à nouveau puis séchées.
Les résultats de teinture suivants ont été obtenus.

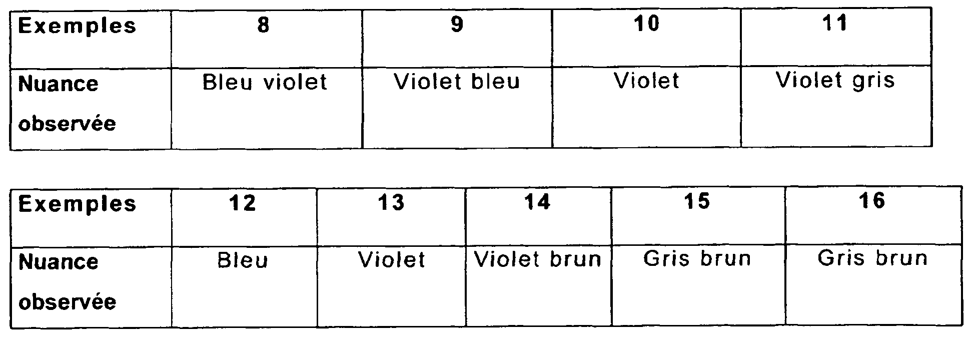

## Revendications

1. Dérivé de paraphénylènediamine substitué par un groupement cyclique diaza de formule (I) ainsi que les sels d'addition correspondant 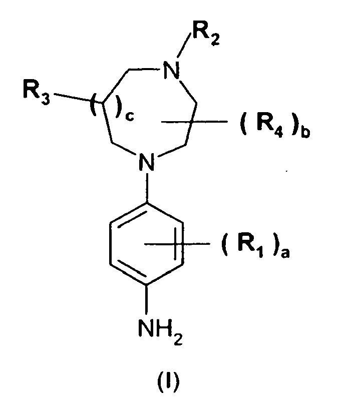 dans laquelle
• **a** est compris entre 0 et 4, étant entendu que lorsque a est supérieur ou égal à 2 alors les radicaux R₁ peuvent être identiques ou différents,
• **b** est compris entre 0 et 4, étant entendu que lorsque b est supérieur ou égal à 2 alors les radicaux R₄ peuvent être identiques ou différents,
• **c** est 0 ou 1,
• **R**_{**1**} représente un atome d'halogène ; une chaîne hydrocarbonée en C₁-C₈, aliphatique ou alicyclique, saturée ou insaturée, un ou plusieurs atomes de carbone pouvant être remplacés par un ou plusieurs atomes d'oxygène, d'azote, de silicium, de soufre ou un groupement SO₂ un radical onium Z ; le radical R₁ ne comportant pas de liaison peroxyde, ni de radicaux diazo, nitro ou nitroso ,
• **R**_{**2**} représente un radical onium Z
• **R**_{**3**} représente
- un radical alkyle ;
- un radical alcényle ;
- un radical alcynyle;
- un radical hydroxy ;
- un radical hydroxyalkyle ;
- un radical alcoxy ;
- un radical alcoxyalkyle ;
- un radical alkylcarbonyle ;
- un radical hydroxyalcoxyalkyle ;
- un radical amino,
- un radical monoalkylamino ou dialkylamino ;
- un radical amino alkyle ;
- un radical aminoalkyle dont l'amine est mono ou disubstituée par un radical alkyle, acétyle ou hydroxyalkyle ;
- un radical hydroxy et amino alkyle ;
- un radical carboxyle ;
- un radical carboxyalkyle ;
- un radical carbamoyle ;
- un radical carbamoylalkyle ;
- un radical alkoxycarbonyle ;
- un radical mono- ou dialkyl- aminocarbonyle ;
- un radical mono- ou dialkyl- aminocarbonylalkyle ;
• **R**_{**4**} représente
- un radical alkyle
- un radical alcényle ;
- un radical alcynyle ;
- un radical hydroxyalkyle ;
- un radical alcoxyalkyle ;
- un radical alkylcarbonyle ;
- un radical hydroxyalcoxyalkyle ;
- un radical aminoalkyle ;
- un radical aminoalkyle dont l'amine est mono ou disubstituée par un radical alkyle, acétyle, hydroxyalkyle ;
- un radical hydroxy et amino alkyle ;
- un radical carboxyle ;
- un radical carboxyalkyle;
- un radical carbamoyle ;
- un radical carbamoylalkyle ;
- un radical alkoxycarbonyle ;
- un radical mono- ou dialkyl- aminocarbonyle ;
- un radical mono- ou dialkyl- aminocarbonylalkyle.

2. Dérivés selon la revendication 1 dans lesquels R₁ est choisi parmi un radical alkyle en C₁-C₄, un radical alcoxyalkyle en C₁-C₄, un radical hydroxyalkyle en C₁₋C₄, un radical aminoalkyle en C₁-C₄, un radical alcoxy en C₁-C₄, un radical hydroxyalcoxy en C₁-C₄, un radical carboxyalkyle en C₁-C₄.

3. Dérivés selon la revendication 2 dans lesquels R₁ est choisi parmi un radical méthyle, hydroxyméthyle, 2-hydroxyéthyle, 1,2-dihydroxyéthyle, méthoxy, isopropyloxy, 2-hydroxyéthoxy.

4. Dérivés selon la revendication 1, 2 ou 3 dans lesquels a est égal à 0 ou 1.

5. Dérivés selon l'une quelconque des revendications 1 à 4 dans lesquels R₂ représente le radical onium Z correspondant à la formule (II) 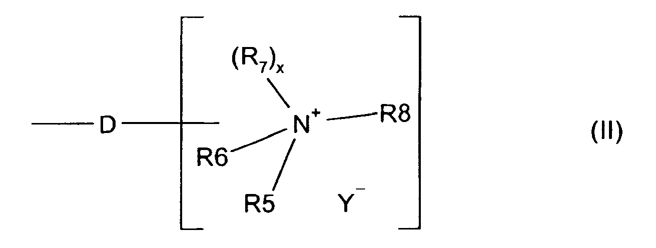 dans laquelle
- D est une liaison covalente ou une chaîne alkylène en C₁-C₁₄, linéaire ou ramifiée pouvant contenir un ou plusieurs hétéroatomes choisis parmi l'oxygène, le soufre ou l'azote, et pouvant être substituée par un ou plusieurs radicaux hydroxyle ou amino, et pouvant comprendre un ou plusieurs carbonyles;
- **R**_{**8**}**, R**_{**5**} et **R**_{**6**}**,** pris séparément, représentent un radical alkyle en C₁-C₁₅ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical carbamoylalkyle en C₁-C₆ ; un radical trialkyl(C₁-C₆)silanealkyle en C₁-C₆ ; un radical aminoalkyle en C₁-C₆ ; un radical aminoalkyle en C₁-C₆ dont l'amine est mono- ou disubstituée par un radical alkyle en C₁-C₄, alkyl(C₁-C₆)carbonyle, carbamoyle ou alkyl(C₁-C₆)sulfonyle ; un radical ammonium quaternaire ;
- **R**_{**8**}**, R**_{**5**} **et R**_{**6**} ensemble, deux à deux, forment, avec l'atome d'azote auxquels ils sont rattachés, un cycle saturé carboné à 4, 5, 6 ou 7 chaînons pouvant contenir un ou plusieurs hétéroatomes, le cycle pouvant être substitué par un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁₋C₆)silanealkyle en C₁-C₆, un radical carbamoyle, un radical carboxyle, un radical alkyl(C₁-C₆)carbonyle, un radical amino, un radical amino mono- ou di-substitué par un radical alkyl(C₁-C₆), alkyl(C₁-C₆)carbonyle, carbamoyle ou alkyl(C₁-C₆)sulfonyle;
- **R**_{**7**} représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆ ; un radical aminoalkyle en C₁-C₆ dont l'amine est mono- ou di-subsitué par un radical alkyl(C₁-C₆), alkyl(C₁-C₆)carbonyle, carbamoyle ou alkyl(C₁₋C₆)sulfonyle ; un radical carboxyalkyle en C₁-C₆ ; un radical carbamoylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ;un radical N-alkyl(C₁₋C₆)carbamoylalkyle en C₁-C₆; un radical N-alkyl(C₁-C₆)sulfonamidoalkyle en C₁-C₆ ;
• x est 0 ou 1,
- lorsque x = 0, alors le bras de liaison est rattaché à l'atome d'azote portant les radicaux R₅, R₆ et R₈
- lorsque x = 1, alors deux des radicaux R₅, R₆ et R₈ forment conjointement avec l'atome d'azote auquel ils sont rattachés un cycle saturé à 4, 5, 6 ou 7 chaînons et D est lié à un atome de carbone du cycle saturé ;
• Y⁻ est un contre-ion.

6. Dérivés selon la revendication 5 dans lesquels x est égal à 0, et R₅ , R₆ et R₈ séparément sont choisis parmi un radical alkyle en C₁-C₆, un radical monohydroxyalkyle en C₁-C₄, un radical polyhydroxyalkyle en C₂-C₄, un radical alcoxy(C₁-C₆)alkyle en C₁-C₄, un radical carbamoylalkyle en C₁-C₆, un radical trialkyl(C₁₋C₆)silanealkyle en C₁-C₆ , ou R₈ avec R₅ forment ensemble un cycle azétidine, pyrrolidine, pipéridine, pipérazine, morpholine, R₆ étant choisi dans ce cas parmi un radical alkyle en C₁-C₆ ;un radical monohydroxyalkyle en C₁-C₆ ; un radical polyhydroxyalkyle en C₂-C₆; un radical aminoalkyle en C₁-C₆, un radical aminoalkyle mono- ou di-substitué par un radical alkyl(C₁-C₆), alkyl(C₁-C₆)carbonyle, carbamoyle ou alkyl(C₁-C₆)sulfonyle ; un radical carbamoylalkyle en C₁-C₆ ; un radical trialkyl(C,-C₆)silanealkyle en C₁-C₆ ; un radical alkyl(C₁-C₆)carboxyalkyle en C₁-C₆ ; un radical alkyl(C₁-C₆)carbonylalkyle en C₁-C₆ ; un radical N-alkyl(C₁-C₆)carbamoylalkyle en C₁-C₆.

7. Dérivés selon la revendication 5 dans lesquels x est égal à 1, R₇ est choisi parmi un radical alkyle en C₁-C₆ ; un radical monohydroxyalkyle en C₁-C₆ ; un radical polyhydroxyalkyle en C₂-C₆ ; un radical aminoalkyle en C₁-C₆, un radical aminoalkyle en C₁-C₆ dont l'amine est mono ou disubstituée par un radical alkyl(C₁-C₆), alkyl(C₁-C₆)carbonyle, carbamoyle ou alkyl(C₁-C₆)sulfonyle ; un radical carbamylalkyle en C₁-C₆ ; un radical trialkyl(C₁-C₆)silanealkyle en C₁-C₆ ; un radical alkyl(C₁₋C₆)carboxyalkyle en C₁-C₆ ; un alkyl(C₁-C₆)carbonylalkyle en C₁-C₆ ; un radical N-alkyl(C₁-C₆)carbamylalkyle en C₁-C₆ ; R₈ avec R₅ ensemble forment un cycle azétidine pyrrolidine, pipéridine, pipérazine, morpholine, R₆ étant choisi dans ce cas parmi un radical alkyle en C₁-C₆ ; un radical monohydroxyalkyle en C₁-C₆ ; un radical polyhydroxyalkyle en C₂-C₆; un radical aminoalkyle en C₁-C₆, un radical aminoalkyle en C₁-C₆ dont l'amine est mono ou disubstituée par un radical alkyl(C₁-C₆), alkyl(C₁₋C₆)carbonyle, carbamoyle ou alkyl(C₁-C₆)suffonyle ; un radical carbamoylalkyle en C₁-C₆ ; un radical trialkyl(C₁-C₆)silanealkyle en C₁-C₆ ; un radical alkyl(C₁-C₆)carboxyalkyle en C₁-C₆ ; un radical alkyl(C₁-C₆)carbonylalkyle en C₁-C₆ ; un radical N-alkyl(C₁₋C₆)carbamoylalkyle en C₁-C₆.

8. Dérivés selon la revendication 5 dans lesquels R₂ est un radical trialkylammoniumalkyle, l'alkyle reliant R₂ au cycle pouvant être substitué par un ou plusieurs groupements hydroxy.

9. Dérivés selon la revendication 5 dans lesquels D est une liaison covalente ou une chaîne alkylène pouvant être substituée et/ou contenir un groupe carbonyle.

10. Dérivés de paraphénylènediamine selon l'une quelconque des revendications 1 à 4 dans lesquels R₂ représente un radical onium Z correspondant à la formule (III) 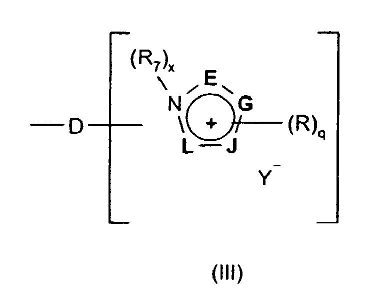 dans laquelle
• D est une liaison covalente ou une chaîne alkylène en C₁-C₁₄, linéaire ou ramifiée pouvant contenir un ou plusieurs hétéroatomes choisis parmi l'oxygène, le soufre ou l'azote, et pouvant être substituée par un ou plusieurs radicaux hydroxyle ou amino, et pouvant comprendre un ou plusieurs carbonyles;
• les sommets E, G, J, L, identiques ou différents, représentent un atome de carbone, d'oxygène, de soufre ou d'azote pour former un cycle pyrrolique, pyrazolique, imidazolique, triazolique, oxazolique, isooxazolique, thiazolique, isothiazolique,
• q est un nombre entier compris entre 1 et 4 ;
• **R,** identiques ou différents, représentent un atome d'hydrogène, un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical carbamoyle, un radical carboxyle, un radical alkylcarbonyle en C₁-C₆, un radical amino, un radical amino mono ou di substituée par un radical alkyl(C₁-C₆), alkyl(C₁-C₆)carbonyle, carbamoyle, alkyl(C₁-C₆)sulfonyle ; un radical monohydroxyalkyle en C₁-C₆ ,un radical polyhydroxyalkyle en C₂-C₆ ou un radical ammonium quaternaire ;
• **R**_{**7**} représente un radical alkyle en C₁-C₆ ; un radical monohydroxyalkyle en C₁₋C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est substitué par un radical alkyl(C₁-C₆), alkyl(C₁-C₆)carbonyle, carbamoyle ou alkyl(C₁-C₆)sulfonyle ; un radical carboxyalkyle en C₁-C₆; un radical carbamoylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁₋C₆)carboxyalkyle en C₁-C₆ ; un radical N-alkyl(C₁-C₆)carbamoylalkyle en C₁-C₆ ; un radical N-alkyl(C₁-C₆)sulfonamidoalkyle en C₁-C₆ ; un radical ammonium quaternaire ;
• x est 0 ou 1
- lorsque x = 0, le bras de liaison D est rattaché à l'atome d'azote,
- lorsque x = 1, le bras de liaison D est rattaché à l'un des sommets E, G, J ou L,
• Y⁻ est un contre-ion.

11. Dérivés selon la revendication 10 dans lesquels les sommets E, G, J et L forment un cycle pyrrolique, imidazolique, pyrazolique, oxazolique, thiazolique et triazolique.

12. Dérivés selon la revendication 11 dans lesquels les sommets E, G, J et L forment un cycle imidazolique.

13. Dérivés selon l'une quelconque des revendications 10 à 12 dans lesquels x est égal à 0, D est une liaison covalente ou une chaîne alkylène pouvant être substituée et/ou contenir une fonction carbonyle.

14. Dérivés de paraphénylènediamine selon l'une quelconque des revendications 1 à 4 dans lesquels R₂ représente un radical onium Z correspondant à la formule (IV) 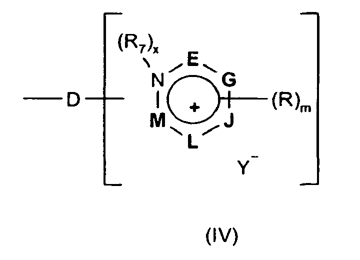 dans laquelle :
• D est une liaison covalente ou une chaîne alkylène en C₁-C₁₄, linéaire ou ramifiée pouvant contenir un ou plusieurs hétéroatomes choisis parmi un atome d'oxygène, de soufre ou d'azote, et pouvant être substituée par un ou plusieurs radicaux hydroxyle ou amino, et pouvant comprendre un ou plusieurs carbonyles ;
• les sommets E, G, J, L et M identiques ou différents, représentent un atome de carbone, d'oxygène, de soufre ou d'azote et forment un cycle choisi parmi les cycles pyridiniques, pyrimidiniques, pyraziniques, triaziniques et pyridaziniques,
• **m** est un nombre entier compris entre 1 et 5 ;
• **R,** identiques ou différents, représentent un atome d'hydrogène, un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical carbamoyle, un radical carboxyle, un radical alkylcarbonyle en C₁-C₆, un radical amino, un radical amino substitué par un radical alkyl(C₁-C₆), alkyl(C₁₋C₆)carbonyle, carbamoyle ou alkyl(C₁-C₆)sulfonyle ; un radical monohydroxyalkyle en C₁-C₆ ou un radical polyhydroxyalkyle en C₂-C₆ ; un radical ammonium quaternaire ;
• **R**_{**7**} représente un radical alkyle en C₁-C₆ ; un radical monohydroxyalkyle en C₁₋C₆ ; un radical polyhydroxyalkyle en C₂-C₆ un radical aryle ; un radical benzyle ; un radical aminoalkyle en C₁-C₆, un radical aminoalkyle en C₁-C₆ dont l'amine est mono- ou di- substituée par un radical alkyl(C₁-C₆), alkyl(C₁-C₆)carbonyle, carbamoyle ou alkyl(C₁-C₆)sulfonyle ; un radical carboxyalkyle en C₁-C₆ ; un radical carbamoylalkyle en C₁-C₆; un radical trifluoroalkyle en C₁-C₆ ; un radical trialkyl(C₁-C₆)silanealkyle en C1-C6; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ un radical alkyl(C₁-C₆)sulfonylalkyle en C₁-C₆; un radical alkyl(C,-C₆)carboxyalkyle en C₁-C₆ un radical N-alkyl(C₁-C₆)carbamoylalkyle en C₁-C₆ ; un radical N-alkyl(C₁-C₆)sulfonamidoalkyle en C₁-C₆ ; ; un radical ammonium quaternaire ;
• **x** est 0 ou 1
- lorsque x = 0, le bras de liaison D est rattaché à l'atome d'azote,
- lorsque x = 1, le bras de liaison D est rattaché à l'un des sommets E, G, J, L ou M,
Y⁻ représente un contre-ion.

15. Dérivés selon la revendication 14 dans lesquels les sommets E,G,J,L et M avec l'azote du cycle forment un cycle choisi parmi les cycles pyridiniques, pyrimidiniques.

16. Dérivés selon l'une quelconque des revendications 10 à 15 dans lesquels x est égal à 0 et **R** est choisi parmi un atome d'hydrogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁₋C₆)silanealkyle en C₁-C₆, un radical carbamoyle, un radical alkylcarbonyle en C₁-C₆, un radical amino, un radical amino mono- ou di-substitué par un radical alkyl(C₁-C₆), alkyl(C₁-C₆)carbonyle, carbamoyle ou alkyl(C₁-C₆)sulfonyle ; un radical monohydroxyalkyle en C₁-C₆ ou un radical polyhydroxyalkyle en C₂-C₆

17. Dérivés selon l'une quelconque des revendications 10 à 15 dans lequel x est égal à 1, **R**₇ est choisi parmi un radical alkyle en C₁-C₆ ; un radical monohydroxyalkyle en C₁-C₆ ; un radical polyhydroxyalkyle en C₂-C₆ ; un radical aminoalkyle en C₁-C₆, un radical aminoalkyle en C₁-C₆ dont l'amine est mono- ou disubstituée par un radical alkyl(C₁-C₆), alkyl(C₁-C₆)carbonyle, un radical carbamoyle ou un radical alkyl(C₁-C₆)sulfonyle ; un radical carbamoylalkyle en C₁-C₆ ; un radical trialkyl(C₁-C₆)silanealkyle en C₁-C₆ ; un radical alkyl(C₁-C₆)carbonylalkyle en C₁-C₆ ; un radical N-alkyl(C₁-C₆)carbamylalkyle en C₁-C₆; **R** est choisi parmi un atome d'hydrogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical carbamoyle, un radical alkylcarbonyle en C₁-C₆, un radical amino, un radical amino mono- ou di-substitué par un radical alkyl(C₁-C₆), alkyl(C₁-C₆)carbonyle, carbamoyle ou alkyl(C₁₋C₆)sulfonyle.

18. Dérivés selon l'une quelconque des revendications 10 à 17 dans lesquels **R** est choisi parmi l'atome d'hydrogène ou des radicaux alkyles pouvant être substitués et R₇ est un radical alkyle pouvant être substitué.

19. Dérivés selon l'une quelconque des revendications 1 à 18 dans lesquels **R** est choisi parmi l'hydrogène ; un radical alkyle ; un radical alkyle substitué par un ou plusieurs hydroxyle ; un radical alkyle substitué par un ou plusieurs amino ; un radical carboxyle, un radical carbamoyle, un radical amino, un radical hydroxyle.

20. Dérivés selon la revendication 19 dans lesquels **R** est choisi parmi l'hydrogène ; le radical hydroxyle; le radical méthyle ; le radical amino ; le radical hydroxyméthyle ; le radical aminométhyle.

21. Dérivés selon l'une quelconque des revendications 1 à 20 dans lesquels b=0 ou **R**_{**4**} est choisi parmi un radical alkyle ; un radical alkyle substitué par un ou plusieurs hydroxy ; un radical alkyle substitué par un ou plusieurs amino ; un radical carboxyle ; un radical carbamoyle.

22. Dérivés selon l'une quelconque des revendications précédentes dans lesquels **R**_{**3**} est choisi parmi l'hydrogène ; le radical hydroxyle ; le radical amino ; un radical alkyle ; un radical alkyle substitué par un ou plusieurs hydroxy ; un radical alkyle substitué par un ou plusieurs amino ; un radical carboxyle ; un radical carbamoyle.

23. Dérivés selon l'une quelconque des revendications 1 à 22 dans lesquels le contre ions Y⁻ est choisi parmi un atome d'halogène, un hydroxyde, un hydrogènesulfate, un acétate, un tartrate ou un alkyl(C₁-C₆)sulfate.

24. Dérivés selon l'une quelconque des revendications précédentes choisis parmi les composés suivants
| | | | |
|---|---|---|---|
| 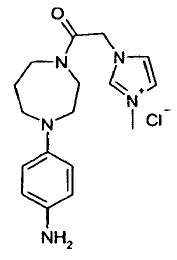 | 3-{2-[4-(4-Amino-phényl)-[1,4]diazépan-1-yl]-2-oxo-éthyl}-1-méthyl-3H-imidazol-1-um ; chlorure | 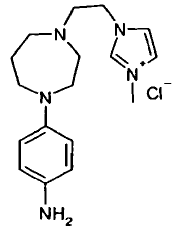 | 3-{2-[4-(4-Amino-phényl)-[1,4]diazépan-1-yl]-éthyl}-1-méthyl-3H-imidazol-1-ium; chlorure |
| 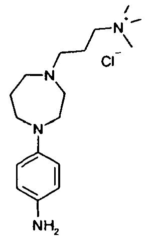 | {3-[4-(4-Amino-phényl)-[1,4]diazépan-1-yl]-propyl}-triméthyl-ammonium; chlorure | 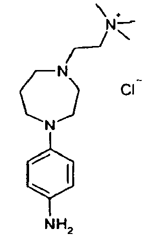 | {2-[4-(4-Amino-phényl)-[1,4]diazépan-1-yl]-éthyl}-triméthyl-ammonium; chlorure |
| 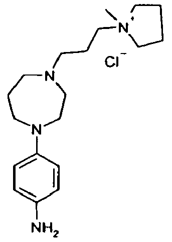 | 1-{3-[4-(4-Amino-phényl)-[1,4]diazépan-1-yl]-propyl}-1-méthyl-pyrrolidinium; chlorure | 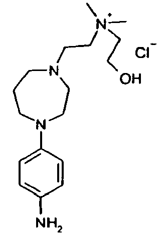 | (2-[4-(4-Amino-phényl)-[1,4]diazépan-1-yl]-éthyl}-(2-hydroxy-éthyl)-diméthyl-ammonium ; chlorure |
| 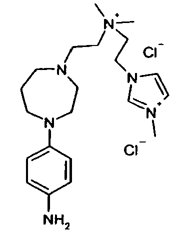 | {3-[4-(4-Amino-phényl)-[1,4]diazépan-1-yl]-éthyl}-diméthyl(éthyl-2--méthyl-3H-imidazol-1-ium chlorure) -ammonium; chlorure | 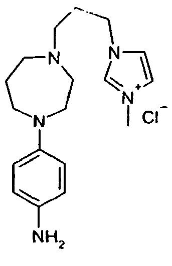 | 3-{2-[4-(4-Amino-phényl)-[1,4]diazépan-1-yl]-propyl}-1-méthyl-3H-imidazol-1-ium; chlorure |
| 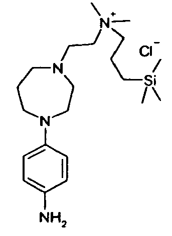 | {2-[4-(4-Amino-phényl)-[1,4]diazépan-1-yl]-éthyl}-diméthyl-(3-triméthylsilanyl-propyl)-ammonium ; chlorure | 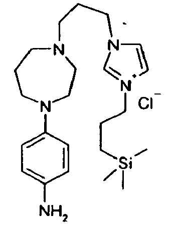 | 3-{2-[4-(4-Amino-phényl)-[1,4]diazépan-1-yl]-éthyl}-1-(3-triméthylsilanyl-propyl)-3H-imidazol-1-ium ; chlorure |
| 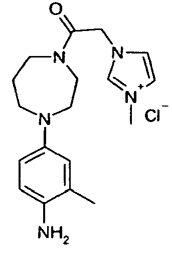 | 3-{2-[4-(4-Amino-3-méthyl-phényl)-[1,4]diazépan-1-yl]-2-oxo-éthyl}-1-méthyl-3H-imidazol-1-um ; chlorure | 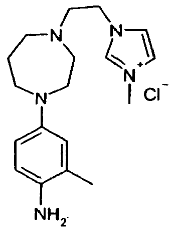 | 3-{2-[4-(4-Amino-3-méthyl-phényl)-[1,4]diazépan-1-yl]-éthyl}-1-méthyl-3H-imidazol-1-um ; chlorure |
| 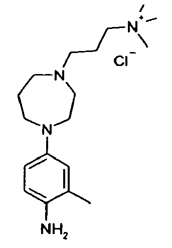 | {3-[4-(4-Amino-3-méthyl-phényl)-[1,4]diazépan-1-yl]-propyl}-triméthyl-ammonium; chlorure | 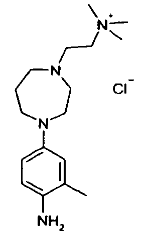 | {2-[4-(4-Amino-3-méthyl-phényl)-[1,4]diazépan-1-yl]-éthyl}-triméthyl-ammonium; chlorure |
| 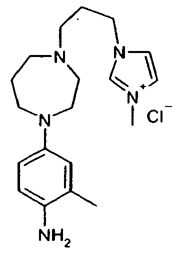 | 3-{2-[4-(4-Amino-3-méthyl-phényl)-[1,4]diazépan-1-yl]-propyl}-1-méthyl-3H-imidazol-1-um ; chlorure | 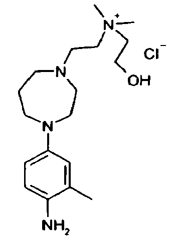 | {2-[4-(4-Amino-3-méthyl-phényl)-[1,4]diazépan-1-yl]-éthyl}-(2-hydroxy-éthyl)-diméthyl-ammonium ; chlorure |
| 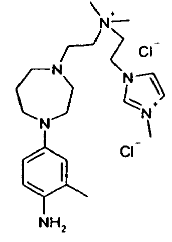 | {3-[4-(4-Amino-3-méthyl-phényl)-[1,4]diazépan-1-yl]-éthyl}-diméthyl(éthyl-2--méthyl-3H-imidazol-1-ium chlorure) -ammonium; chlorure | 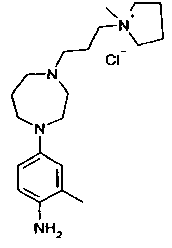 | 1-{3-[4-(4-Amino-3-méthyl-phényl)-[1,4]diazépan-1-yl]-propyl}-1-méthyl-pyrrolidinium ; chlorure |
| 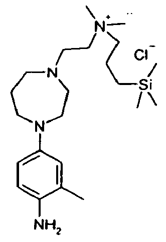 | {2-[4-(4-Amino-3-méthyl-phényl)-[1,4]diazépan-1-yl]-éthyl}-diméthyl-(3-triméthylsilanyl-propyl)-ammonium ; chlorure | 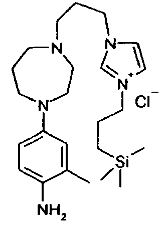 | 3-{2-[4-(4-Amino-3-méthyl-phényl)-[1,4]diazépan-1-yl]-éthyl}-1-(3-triméthylsilanyl-propyl)-3H-imidazol-1-ium ; chlorure |
| 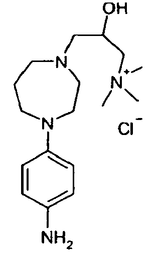 | {3-[4-(4-Amino-phényl)-[1,4]diazepan-1-yl]-2-hydroxy-propyl}-triméthyl-ammonium; chlorure | 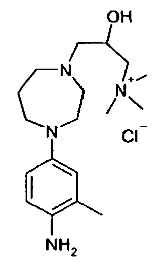 | {3-[4-(4-Amino-3-méthyl-phényl)-[1,4]diazépan-1-yl]-2-hydroxy-propyl}-triméthyl-ammonium; chlorure |
| 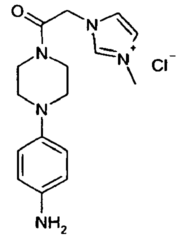 | 3-{2-[4-(4-Amino-phényl)-piperazin-1-yl]-2-oxo-éthyl}-1-méthyl-3H-imidazol-1-ium; chlorure | 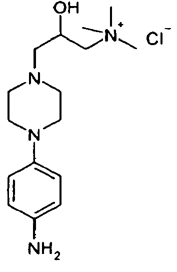 | {3-[4-(4-Amino-phényl)-piperazin-1-yl]-2-hydroxy-propyl}-triméthyl-ammonium; chlorure |
| 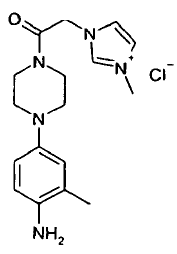 | Chlorure de 3-{2-[4-(4-Amino-3-méthyl-phényl)-piperazin-1-yl]-2-oxo-éthyl}-1-méthyll-3H-imidazol-1-ium; | 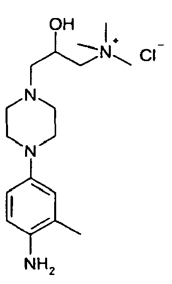 | Chlorure de 3-[4-(4-Amino-3-méthyl-phényl)-piperazin-1-yl]-2-hydroxy-propyl}-triméthyl-ammonium; |
| 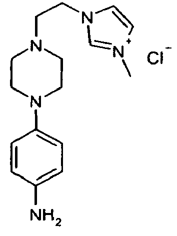 | 3-{2-[4-(4-Amino-phényl)-piperazin-1-yl]-éthyl}-1-méthyl-3H-imidazol-1-ium; chlorure | 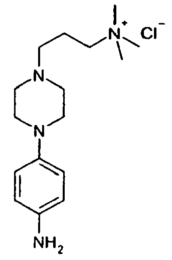 | {3-[4-(4-Amino-phényl)-piperazin-1-yl]-propyl}-triméthyl-ammonium; chloride |
| 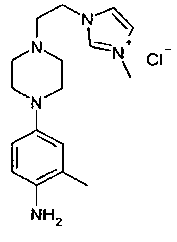 | 3-{2-[4-(4-Amino-3-méthyl-phényl)-piperazin-1-yl]-éthyl}-1-méthyl-3H-imidazol-1-ium; chlorure | 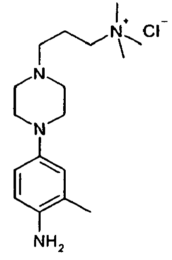 | {3-[4-(4-Amino-3-méthyl-phényl)-piperazin-1-yl]-propyl}-triméthyl-ammonium; chlorure |
| 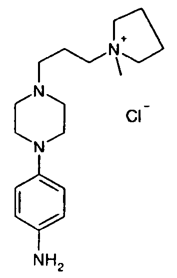 | 1-{3-[4-(4-Amino-phényl)-piperazin-1-yl]-propyl}-1-méthyl-pyrrolidinium; chlorure | | |
| 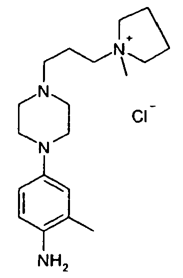 | 1-{3-[4-(4-Amino-3-méthyl-phényl)-piperazin-1-yl]-propyl}-1-méthyl-pyrrolidinium; chlorure | | |

25. Dérivés nitro de formule (I') suivantes 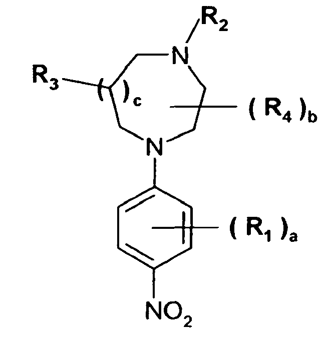 dans laquelle R₁, R₂, R₃, R₄, a, b, c sont tels que définis selon l'une quelconque des revendications 1 à 24.

26. Composition tinctoriale comprenant à titre de base d'oxydation au moins un dérivé de para-phénylènediamine de formule (I) tel que défini selon l'une quelconque des revendications 1 à 24.

27. Composition selon la revendication 26 comprenant de plus un coupleur choisi parmi les méta-phénylènediamines, les méta-aminophénols, les méta-diphénols, les coupleurs naphtaléniques, les coupleurs hétérocycliques et leurs sels d'addition

28. Composition selon l'une quelconque des revendications 26 ou 27 comprenant une base d'oxydation additionnelle autre que les bases d'oxydation de formule (I) choisie parmi les para-phénylènediamines, les bis-phénylalkylènediamines, les para-aminophénols, les ortho-aminophénols, les bases hétérocycliques et leurs sels d'addition.

29. Composition selon l'une quelconque des revendications 26 à 28 dans laquelle la quantité de chacune des bases d'oxydation est comprise entre 0,001 à 10 % en poids environ du poids total de la composition tinctoriale.

30. Composition selon la revendication 29 dans laquelle la quantité de chacun des coupleurs est comprise entre 0,001 et 10 % en poids environ du poids total de la composition tinctoriale.

31. Composition selon l'une quelconque des revendications 26 à 30 comprenant un milieu cosmétique approprié à la teinture des fibres kératiniques.

32. Procédé de teinture d'oxydation des fibres kératiniques, **caractérisé en ce qu'**on applique sur les fibres une composition tinctoriale telle que définie dans l'une quelconque des revendications 26 à 31 en présence d'un agent oxydant pendant un temps suffisant pour développer la coloration désirée.

33. Procédé selon la revendication 32 dans lequel l'agent oxydant est choisi parmi le peroxyde d'hydrogène, le peroxyde d'urée, les bromates de métaux alcalins, les persels, les peracides et les enzymes oxydases.

34. Dispositif à plusieurs compartiments dans lequel un premier compartiment contient une composition tinctoriale telle que définie à l'une quelconque des revendications 26 à 31 et un deuxième compartiment contient un agent oxydant.

35. Utilisation de la composition définie aux revendications 26 à 31 pour la teinture de fibres kératiniques.

## Claims

1. para-Phenylenediamine derivative substituted with a cyclic diaza group of formula (I), and also the corresponding addition salts 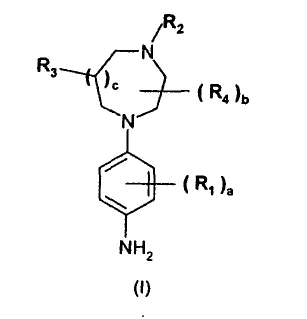 in which
• **a** is between 0 and 4, it being understood that when a is greater than or equal to 2, then the radicals R₁ may be identical or different,
• **b** is between 0 and 4, it being understood that when b is greater than or equal to 2, then the radicals R₄ may be identical or different,
• **c** is 0 or 1,
• **R**_{**1**} represents a halogen atom; a C₁-C₈ aliphatic or alicyclic, saturated or unsaturated hydrocarbon-based chain, one or more carbon atoms possibly being replaced with one or more oxygen, nitrogen, silicon or sulphur atoms or an SO₂ group; an onium radical Z; the radical R₁ not comprising a peroxide bond or diazo, nitro or nitroso radicals,
• **R**_{**2**} represents an onium radical Z
• **R**_{**3**} represents
- an alkyl radical;
- an alkenyl radical;
- an alkynyl radical;
- a hydroxyl radical;
- a hydroxyalkyl radical;
- an alkoxy radical;
- an alkoxyalkyl radical;
- an alkylcarbonyl radical;
- a hydroxyalkoxyalkyl radical;
- an amino radical;
- a monoalkylamino or dialkylamino radical;
- an aminoalkyl radical;
- an aminoalkyl radical in which the amine is monosubstituted or disubstituted with an alkyl, acetyl or hydroxyalkyl radical;
- a hydroxyl and aminoalkyl radical;
- a carboxyl radical;
- a carboxyalkyl radical;
- a carbamoyl radical;
- a carbamoylalkyl radical;
- an alkoxycarbonyl radical;
- a monoalkylaminocarbonyl or dialkylaminocarbonyl radical;
- a monoalkylaminocarbonylalkyl or dialkylamino-carbonylalkyl radical;
• **R**_{**4**} represents
- an alkyl radical;
- an alkenyl radical;
- an alkynyl radical;
- a hydroxyalkyl radical;
- an alkoxyalkyl radical;
- an alkylcarbonyl radical;
- a hydroxyalkoxyalkyl radical;
- an aminoalkyl radical;
- an aminoalkyl radical in which the amine is monosubstituted or disubstituted with an alkyl, acetyl or hydroxyalkyl radical;
- a hydroxyl and aminoalkyl radical;
- a carboxyl radical;
- a carboxyalkyl radical;
- a carbamoyl radical;
- a carbamoylalkyl radical;
- an alkoxycarbonyl radical;
- a monoalkylaminocarbonyl or dialkylaminocarbonyl radical;
- a monoalkylaminocarbonylalkyl or dialkylamino-carbonylalkyl radical.

2. Derivatives according to Claim 1, in which R₁ is chosen from a C₁-C₄ alkyl radical, a C₁-C₄ alkoxyalkyl radical, a C₁-C₄ hydroxyalkyl radical, a C₁-C₄ aminoalkyl radical, a C₁-C₄ alkoxy radical, a C₁-C₄ hydroxyalkoxy radical and a C₁-C₄ carboxyalkyl radical.

3. Derivatives according to Claim 2, in which R₁ is chosen from a methyl, hydroxymethyl, 2-hydroxyethyl, 1,2-dihydroxyethyl, methoxy, isopropyloxy or 2-hydroxyethoxy radical.

4. Derivatives according to Claim 1, 2 or 3, in which **a** is equal to 0 or 1.

5. Derivatives according to any one of Claims 1 to 4, in which R₂ represents the onium radical Z corresponding to formula (II) 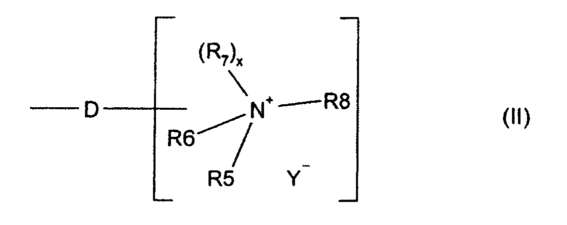 in which
- D is a covalent bond or a linear or branched C₁-C₁₄ alkylene chain which may contain one or more hetero atoms chosen from oxygen, sulphur and nitrogen, and which may be substituted with one or more hydroxyl or amino radicals, and which may comprise one or more carbonyls;
- **R**_{**8**}**, R**_{**5**} and **R**_{**6**}**,** taken separately, represent a C₁-C₁₅ alkyl radical; a C₁-C₆ monohydroxyalkyl radical; a C₂-C₆ polyhydroxyalkyl radical; a (C₁-C₆)alkoxy-(C₁-C₆)alkyl radical; an aryl radical; a benzyl radical; a C₁-C₆ carbamoylalkyl radical; a tri(C₁₋C₆)alkylsilane(C₁-C₆) alkyl radical; a C₁-C₆ aminoalkyl radical; a C₁-C₆ aminoalkyl radical in which the amine is mono- or disubstituted with a C₁-C₄ alkyl, (C₁-C₆) alkylcarbonyl, carbamoyl or (C₁₋C₆)alkylsulphonyl radical; a quaternary ammonium radical;
- **R**_{**8**}**, R**_{**5**} and **R**_{**6**} together, in pairs, form, with the nitrogen atom to which they are attached, a saturated 4-, 5-, 6- or 7-membered carbon-based ring optionally containing one or more hetero atoms, the ring possibly being substituted with a hydroxyl radical, a C₁-C₆ alkyl radical, a C₁-C₆ monohydroxyalkyl radical, a C₂-C₆ polyhydroxyalkyl radical, a C₁-C₆ alkoxy radical, a tri (C₁-C₆) alkylsilane(C₁-C₆)alkyl radical, a carbamoyl radical, a carboxyl radical, a (C₁-C₆)alkylcarbonyl radical, an amino radical, an amino radical mono- or disubstituted with a (C₁-C₆)alkyl, (C₁₋C₆)alkylcarbonyl, carbamoyl or (C₁₋C₆)alkylsulphonyl radical;
- **R**_{**7**} represents a C₁-C₆ alkyl radical; a C₁-C₆ monohydroxyalkyl radical; a C₂-C₆ polyhydroxyalkyl radical; an aryl radical; a benzyl radical; a C₁-C₆ aminoalkyl radical; a C₁-C₆ aminoalkyl radical in which the amine is mono- or disubstituted with a (C₁-C₆)alkyl, (C₁-C₆)alkylcarbonyl, carbamoyl or (C₁-C₆)alkylsulphonyl radical; a C₁-C₆ carboxyalkyl radical; a C₁-C₆ carbamoylalkyl radical; a C₁-C₆ trifluoroalkyl radical; a tri(C₁₋C₆)alkylsilane (C₁-C₆)alkyl radical; a C₁-C₆ sulphonamidoalkyl radical; a (C₁₋C₆)alkylcarboxy(C₁-C₆)alkyl radical; a (C₁-C₆)-alkylsulphinyl (C₁-C₆)alkyl radical; a (C₁-C₆)-alkylsulphonyl (C₁-C₆)alkyl radical; a (C₁-C₆)alkylcarbonyl (C₁-C₆)alkyl radical; an N- (C₁-C₆)alkylcarbamoyl (C₁-C₆)alkyl radical; an N-(C₁-C₆)alkyl-sulphonamido(C₁-C₆)alkyl radical;
• x is 0 or 1,
- when x = 0, then the linker arm is attached to the nitrogen atom bearing the radicals R₅, R₆ and R₈,
- when x = 1, then two of the radicals R₅, R₆ and R₈ form, together with the nitrogen atom to which they are attached, a 4-, 5-, 6- or 7-membered saturated ring and D is linked to a carbon atom of the saturated ring;
• Y⁻ is a counterion.

6. Derivatives according to Claim 5, in which x is equal to 0, and R₅, R₆ and R₈ are separately chosen from a C₁-C₆ alkyl radical, a C₁-C₄ monohydroxyalkyl radical, a C₂-C₄ polyhydroxyalkyl radical, a (C₁-C₆) alkoxy (C₁-C₄)-alkyl radical, a C₁-C₆ carbamoyl alkyl radical, a tri(C₁-C₆)alkylsilane(C₁-C₆)alkyl radical, or R₈ and R₅ together form an azetidine, pyrrolidine, piperidine, piperazine or morpholine ring, R₆ being chosen in this case from a C₁-C₆ alkyl radical; a C₁-C₆ monohydroxyalkyl radical; a C₂-C₆ polyhydroxyalkyl radical; a C₁-C₆ aminoalkyl radical, an aminoalkyl radical mono- or disubstituted with a (C₁-C₆)alkyl, (C₁-C₆)alkylcarbonyl, carbamoyl or (C₁-C₆)alkylsulphonyl radical; a C₁-C₆ carbamoylalkyl radical; a tri(C₁-C₆)alkylsilane(C₁₋C₆)alkyl radical; a (C₁-C₆) alkylcarboxy (C₁-C₆)alkyl radical; a (C₁-C₆) alkylcarbonyl(C₁-C₆)alkyl radical; an N-(C₁-C₆) alkylcarbamoyl (C₁-C₆)alkyl radical.

7. Derivatives according to Claim 5, in which x is equal to 1, and R₇ is chosen from a C₁-C₆ alkyl radical; a C₁-C₆ monohydroxyalkyl radical; a C₂-C₆ polyhydroxyalkyl radical; a C₁-C₆ aminoalkyl radical; a C₁-C₆ aminoalkyl radical in which the amine is mono- or disubstituted with a (C₁-C₆) alkyl, (C₁-C₆) alkylcarbonyl, carbamoyl or (C₁-C₆)alkylsulphonyl radical; a C₁-C₆ carbamylalkyl radical; a tri (C₁-C₆) alkylsilane (C₁₋C₆) alkyl radical; a (C₁-C₆) alkylcarboxy (C₁-C₆) alkyl radical; a (C₁-C₆)alkylcarbonyl(C₁-C₆)alkyl radical; an N- (C₁-C₆) alkylcarbamyl (C₁-C₆) alkyl radical; R₈ and R₅ together form an azetidine, pyrrolidine, piperidine, piperazine or morpholine ring, R₆ being chosen in this case from a C₁-C₆ alkyl radical; a C₁-C₆ monohydroxyalkyl radical; a C₂-C₆ polyhydroxyalkyl radical; a C₁-C₆ aminoalkyl radical, a C₁-C₆ aminoalkyl radical in which the amine is mono- or disubstituted with a (C₁-C₆)alkyl, (C₁-C₆) alkylcarbonyl, carbamoyl or (C₁-C₆)alkylsulphonyl radical; a C₁-C₆ carbamoylalkyl radical; a tri(C₁₋C₆)alkylsilane (C₁-C₆) alkyl radical; a (C₁₋C₆) alkylcarboxy (C₁-C₆) alkyl radical; a (C₁-C₆)alkylcarbonyl (C₁-C₆) alkyl radical; an N-(C₁-C₆)alkylcarbamoyl-(C₁-C₆)alkyl radical.

8. Derivatives according to Claim 5, in which R₂ is a trialkylammonium alkyl radical, the alkyl linking R₂ to the ring possibly being substituted with one or more hydroxyl groups.

9. Derivatives according to Claim 5, in which D is a covalent bond or an alkylene chain that may be substituted and/or that may contain a carbonyl group.

10. para-Phenylenediamine derivatives according to any one of Claims 1 to 4, in which R₂ represents an onium radical Z corresponding to formula (III) 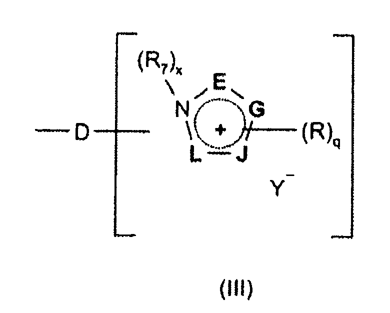 in which
• D is a covalent bond or a linear or branched C₁-C₁₄ alkylene chain that may contain one or more hetero atoms chosen from oxygen, sulphur and nitrogen, and that may be substituted with one or more hydroxyl or amino radicals, and that may comprise one or more carbonyls;
• the ring members E, G, J and L, which may be identical or different, represent a carbon, oxygen, sulphur or nitrogen atom to form a pyrrole, pyrazole, imidazole, triazole, oxazole, isooxazole, thiazole or isothiazole ring,
• q is an integer between 1 and 4;
• **R,** which may be identical or different, represent a hydrogen atom, a halogen atom, a hydroxyl radical, a C₁-C₆ alkyl radical, a C₁-C₆ monohydroxyalkyl radical, a C₂-C₆ polyhydroxyalkyl radical, a C₁-C₆ alkoxy radical, a tri(C₁₋C₆) alkylsilane (C₁-C₆) alkyl radical, a carbamoyl radical, a carboxyl radical, a C₁-C₆ alkylcarbonyl radical, an amino radical, an amino radical mono- or disubstituted with a (C₁-C₆) alkyl, (C₁₋C₆)alkylcarbonyl, carbamoyl or (C₁-C₆)alkylsulphonyl radical; a C₁-C₆ monohydroxyalkyl radical or a C₂-C₆ polyhydroxyalkyl radical, or a quaternary ammonium radical;
• **R**_{**7**} represents a C₁-C₆ alkyl radical; a C₁-C₆ monohydroxyalkyl radical; a C₂-C₆ polyhydroxyalkyl radical; an aryl radical; a benzyl radical; a C₁-C₆ aminoalkyl radical, a C₁-C₆ aminoalkyl radical in which the amine is substituted with a (C₁-C₆)alkyl, (C₁-C₆)alkylcarbonyl, carbamoyl or (C₁₋C₆)alkylsulphonyl radical; a C₁-C₆ carboxyalkyl radical; a C₁-C₆ carbamoylalkyl radical; a C₁-C₆ trifluoroalkyl radical; a tri(C₁-C₆)alkylsilane(C₁₋C₆)alkyl radical; a C₁-C₆ sulphonamidoalkyl radical; a (C₁-C₆) alkylcarboxy (C₁-C₆) alkyl radical; a (C₁-C₆)alkylsulphinyl(C₁-C₆)alkyl radical; a (C₁₋C₆)alkylsulphonyl(C₁-C₆)alkyl radical; a (C₁₋C₆)alkylcarboxy(C₁-C₆)alkyl radical; an N-(C₁₋C₆)alkylcarbamoyl (C₁-C₆)alkyl radical; an N- (C₁₋C₆)alkylsulphonamido (C₁-C₆)alkyl radical; a quaternary ammonium radical;
• x is 0 or 1
- when x = 0, the linker arm D is attached to the nitrogen atom,
- when x = 1, the linker arm D is attached to one of the ring members E, G, J or L,
• Y⁻ is a counterion.

11. Derivatives according to Claim 10, in which the ring members E, G, J and L form a pyrrole, imidazole, pyrazole, oxazole, thiazole or triazole ring.

12. Derivatives according to Claim 11, in which the ring members E, G, J and L form an imidazole ring.

13. Derivatives according to any one of Claims 10 to 12, in which x is equal to 0 and D is a covalent bond or an alkylene chain that may be substituted and/or that may contain a carbonyl function.

14. para-Phenylenediamine derivatives according to any one of Claims 1 to 4, in which R₂ represents an onium radical Z corresponding to formula (IV) 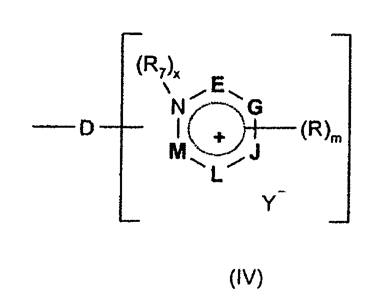 in which:
• D is a covalent bond or a linear or branched C₁-C₁₄ alkylene chain which may contain one or more hetero atoms chosen from oxygen, sulphur and nitrogen atoms, and which may be substituted with one or more hydroxyl or amino radicals, and that may comprise one or more carbonyls;
• the ring members E, G, J, L and M, which may be identical or different, represent a carbon, oxygen, sulphur or nitrogen atom and form a ring chosen from pyridine, pyrimidine, pyrazine, triazine and pyridazine rings;
• **m** is an integer between 1 and 5;
• **R,** which may be identical or different, represent a hydrogen atom, halogen atom, a hydroxyl radical, a C₁-C₆ alkyl radical, a C₁-C₆ monohydroxyalkyl radical, a C₂-C₆ polyhydroxyalkyl radical, a C₁-C₆ alkoxy radical, a tri(C₁-C₆)alkylsilane(C₁-C₆)alkyl radical, a carbamoyl radical, a carboxyl radical, a C₁-C₆ alkylcarbonyl radical, an amino radical, an amino radical substituted with a (C₁-C₆)alkyl, (C₁₋C₆)alkylcarbonyl, carbamoyl or (C₁-C₆)alkylsulphonyl radical; a C₁-C₆ monohydroxyalkyl radical or a C₂-C₆ polyhydroxyalkyl radical; a quaternary ammonium radical;
• **R**_{**7**} represents a C₁-C₆ alkyl radical; a C₁-C₆ monohydroxyalkyl radical; a C₂-C₆ polyhydroxyalkyl radical; an aryl radical; a benzyl radical; a C₁-C₆ aminoalkyl radical; a C₁-C₆ aminoalkyl radical in which the amine is mono- or disubstituted with a (C₁-C₆)alkyl, (C₁-C₆)alkylcarbonyl, carbamoyl or (C₁-C₆)alkylsulphonyl radical; a C₁-C₆ carboxyalkyl radical; a C₁-C₆ carbamoylalkyl radical; a C₁-C₆ trifluoroalkyl radical; a tri(C₁-C₆)alkylsilane(C₁₋C₆)alkyl radical; a C₁-C₆ sulphonamidoalkyl radical; a (C₁-C₆)alkylcarboxy(C₁-C₆)alkyl radical; a (C₁-C₆)alkylsulphinyl(C₁-C₆)alkyl radical; a (C₁₋C₆) alkylsulphonyl (C₁-C₆)alkyl radical; a (C₁₋C₆)alkylcarboxy(C₁-C₆)alkyl radical; an N-(C₁₋C₆) alkylcarbamoyl (C₁-C₆)alkyl radical; an N- (C₁₋C₆)alkylsulphonamido(C₁-C₆)alkyl radical; a quaternary ammonium radical;
• **x** is 0 or 1
- when x = 0, the linker arm D is attached to the nitrogen atom,
- when x = 1, the linker arm D is attached to one of the ring members E, G, J, L or M,
• Y⁻ is a counterion.

15. Derivatives according to Claim 14, in which the ring members E, G, J, L and M form, with the nitrogen of the ring, a ring chosen from pyridine and pyrimidine rings.

16. Derivatives according to any one of Claims 10 to 15, in which x is equal to 0 and **R** is chosen from a hydrogen atom, a hydroxyl radical, a C₁-C₆ alkyl radical, a C₁-C₆ monohydroxyalkyl radical, a C₂-C₆ polyhydroxyalkyl radical, a C₁-C₆ alkoxy radical, a tri(C₁-C₆)alkylsilane(C₁-C₆)alkyl radical, a carbamoyl radical, a C₁-C₆ alkylcarbonyl radical, an amino radical, an amino radical mono- or disubstituted with a (C₁-C₆) alkyl, (C₁-C₆) alkylcarbonyl, carbamoyl or (C₁₋C₆)alkylsulphonyl radical; a C₁-C₆ monohydroxyalkyl radical or a C₂-C₆ polyhydroxyalkyl radical.

17. Derivatives according to any one of Claims 10 to 15, in which x is equal to 1, **R**₇ is chosen from a C₁-C₆ alkyl radical; a C₁-C₆ monohydroxyalkyl radical; a C₂-C₆ polyhydroxyalkyl radical; a C₁-C₆ aminoalkyl radical, a C₁-C₆ aminoalkyl radical in which the amine is mono- or disubstituted with a (C₁-C₆)alkyl radical, a (C₁₋C₆)alkylcarbonyl radical, a carbamoyl radical or a (C₁₋C₆)alkylsulphonyl radical; a C₁-C₆ carbamoylalkyl radical; a tri(C₁-C₆)alkylsilane(C₁-C₆) alkyl radical; a (C₁-C₆) alkylcarbonyl (C₁-C₆) alkyl radical; an N- (C₁₋C₆)alkylcarbamyl(C₁-C₆)alkyl radical; **R** is chosen from a hydrogen atom, a hydroxyl radical, a C₁-C₆ alkyl radical, a C₁-C₆ monohydroxyalkyl radical, a C₂-C₆ polyhydroxyalkyl radical, a C₁-C₆ alkoxy radical, a tri(C₁-C₆)alkylsilane(C₁-C₆)alkyl radical, a carbamoyl radical, a C₁-C₆ alkylcarbonyl radical, an amino radical, an amino radical mono- or disubstituted with a (C₁-C₆) alkyl, (C₁-C₆) alkylcarbonyl, carbamoyl or (C₁-C₆) - alkylsulphonyl radical.

18. Derivatives according to any one of Claims 10 to 17, in which **R** is chosen from a hydrogen atom and alkyl radicals that may be substituted, and R₇ is an alkyl radical that may be substituted.

19. Derivatives according to any one of Claims 1 to 18, in which **R** is chosen from hydrogen; an alkyl radical; an alkyl radical substituted with one or more hydroxyl; an alkyl radical substituted with one or more amino; a carboxyl radical; a carbamoyl radical; an amino radical; a hydroxyl radical.

20. Derivatives according to Claim 19, in which **R** is chosen from hydrogen; a hydroxyl radical; a methyl radical; an amino radical; a hydroxymethyl radical; an aminomethyl radical.

21. Derivatives according to any one of Claims 1 to 20, in which b=0 or **R**_{**4**} is chosen from an alkyl radical; an alkyl radical substituted with one or more hydroxyl; an alkyl radical substituted with one or more amino; a carboxyl radical; a carbamoyl radical.

22. Derivatives according to any one of the preceding claims, in which **R**_{**3**} is chosen from hydrogen; a hydroxyl radical; an amino radical; an alkyl radical; an alkyl radical substituted with one or more hydroxyl; an alkyl radical substituted with one or more amino; a carboxyl radical; a carbamoyl radical.

23. Derivatives according to any one of Claims 1 to 22, in which counterion Y⁻ is chosen from a halogen atom, a hydroxide, a hydrogen sulphate, an acetate, a tartrate and a (C₁-C₆)alkyl sulphate.

24. Derivatives according to any one of the preceding claims, chosen from the following compounds
| | | | |
|---|---|---|---|
| 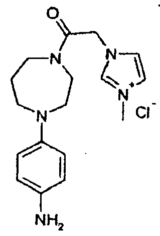 | 3-{2-[4-(4-Amino-phenyl)[1,4]-diazepan-1-yl]-2-oxoethyl}-1-methyl-3H-imidazol-1-ium chloride | 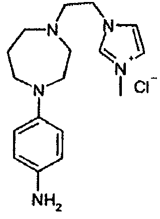 | 3-{2-[4-(4-Amino-phenyl)[1,4]-diazepan-1-yl]ethyl}-1-methyl-3H-imidazol-1-ium chloride |
| 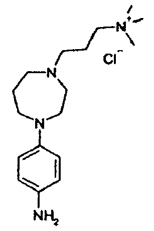 | {3-[4-(4-Amino-phenyl)[1,4]-diazepan-1-yl]propyl}-trimethylammonium chloride | 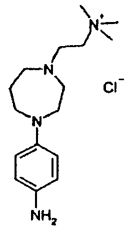 | {2-[4-(4-Amino-phenyl)[1,4]-diazepan-1-yl]ethyl}-trimethylammonium chloride |
| 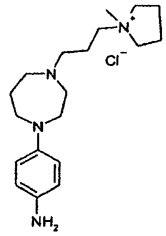 | 1-{3-[4-(4-Amino-phenyl)[1,4]-diazepan-1-yl]propyl}-1-methyl-pyrrolidinium chloride | 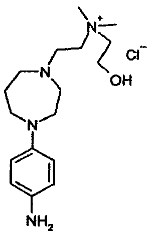 | {2-[4-(4-Amino-phenyl) [1,4]-diazepan-1-yl]ethyl}-(2-hydroxyethyl)-dimethylammonium chloride |
| 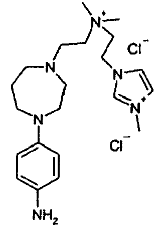 | {3-[4-(4-Amino-phenyl) [1,4]-diazepan-1-yl]ethyl}dimethyl (ethyl-2-methyl-3H-imidazol-1-ium chloride)ammonium chloride | 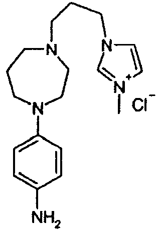 | 3-{2-[4-(4-Amino-phenyl)[1,4]-diazepan-1-yl]propyl}-1-methyl-3H-imidazol-1-ium chloride |
| 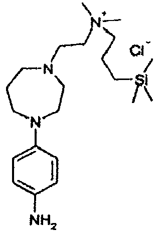 | {2-[4-(4-Amino-phenyl)[1,4]-diazepan-1-yl]ethyl}-dimethyl-(3-trimethylsilanyl-propyl)ammonium chloride | 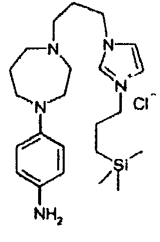 | 3-{2-[4-(4-Amino-phenyl)[1,4]-diazepan-1-yl]ethyl}-1-(3-trimethylsilanyl-propyl)-3H-imidazol-1-ium chloride |
| 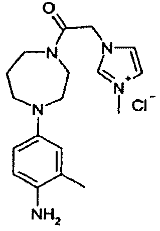 | 3-{2-[4-(4-Amino-3-methylphenyl)-[1,4]diazepan-1-yl]-2-oxoethyl}-1-methyl-3H-imidazol-1-ium chloride | 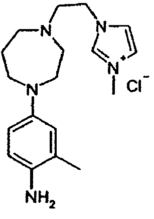 | 3-{2-[4-(4-Amino-3-methylphenyl)-[1,4]diazepan-1-yl]ethyl}-1-methyl-3H-imidazol-1-ium chloride |
| 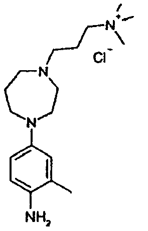 | {3-[4-(4-Amino-3-methylphenyl)-[1,4]diazepan-1-yl]-propyl}trimethyl-ammonium chloride | 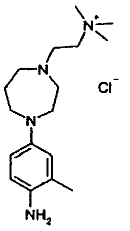 | {2-[4-(4-Amino-3-methylphenyl)-[1,4]diazepan-1-yl]ethyl}-trimethylammonium chloride |
| 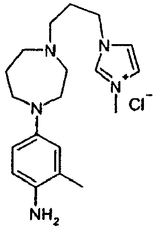 | 3-{2-[4-(4-Amino-3-methylphenyl)-[1,4]diazepan-1-yl]propyl}-1-methyl-3H-imidazol-1-ium chloride | 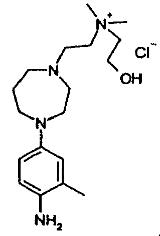 | {2-[4-(4-Amino-3-methylphenyl)-[1,4]diazepan-1-yl]ethyl}(2-hydroxyethyl)-dimethylammonium chloride |
| 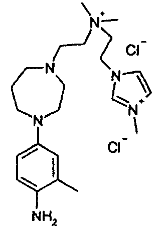 | {3-[4-(4-Amino-3-methylphenyl)-[1,4]diazepan-1-yl]ethyl}dimethyl (ethyl-2-methyl-3H-imidazol-1-ium chloride)ammonium chloride | 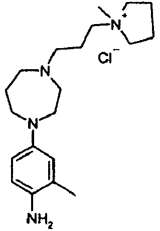 | 1-{3-[4-(4-Amino-3-methylphenyl)-[1,4]diazepan-1-yl]propyl}-1-methyl-pyrrolidinium chloride |
| 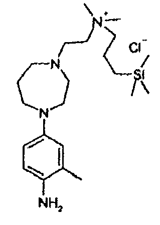 | {2-[4-(4-Amino-3-methylphenyl)-[1,4]diazepan-1-yl]ethyl}-dimethyl(3-trimethyl-silanylpropyl)-ammonium chloride | 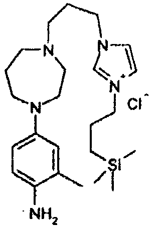 | 3-{2-[4-(4-Amino-3-methylphenyl)-[1,4]diazepan-1-yl]ethyl}-1-(3-trimethyl-silanylpropyl)-3H-imidazol-1-ium chloride |
| 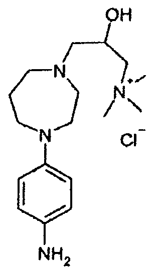 | {3-[4-(4-Amino-phenyl)[1,4]-diazepan-1-yl]-2-hydroxypropyl}-trimethylammonium chloride | 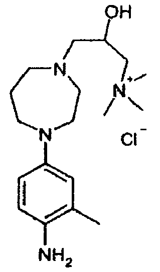 | {3-[4-(4-Amino-3-methylphenyl)-[1,4-diazepan-1-yl]-2-hydroxy-propyl}tri-methylammonium chloride |
| 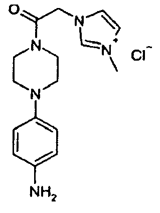 | 3-{2-[4-(4-Amino-phenyl)piperazin-1-yl]-2-oxoethyl}-1-methyl-3H-imidazol-1-ium chloride | 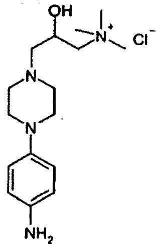 | {3-[4-(4-Amino-phenyl)piperazin-1-yl]-2-hydroxypropyl}-trimethylammonium chloride |
| 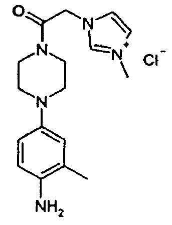 | 3-{2-[4-(4-Amino-3-methylphenyl)-piperazin-1-yl]-2-oxoethyl}-1-methyl-3H-imidazol-1-ium chloride | 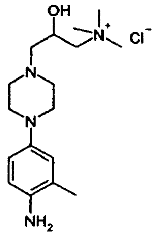 | 3-[4-(4-Amino-3-methylphenyl)-piperazin-1-yl]-2-hydroxypropyl}-trimethylammonium chloride |
| 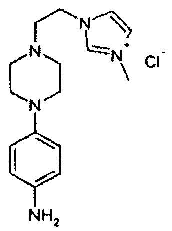 | 3-{2-[4-(4-Amino-phenyl)piperazin-1-yl]ethyl}-1-methyl-3H-imidazol-1-ium chloride | 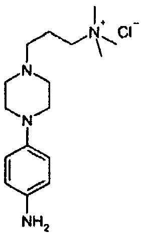 | {3-[4-(4-Amino-phenyl)piperazin-1-yl]propyl}-trimethylammonium chloride |
| 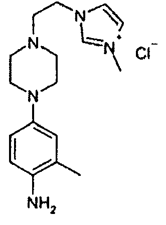 | 3-{2-(4-(4-Amino-3-methylphenyl)-piperazin-1-yl]-ethyl}-1-methyl-3H-imidazol-1-ium chloride | 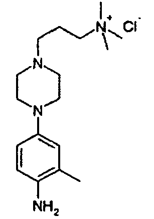 | {3-[4-(4-Amino-3-methylphenyl)-piperazin-1-yl]-propyl}trimethyl-ammonium chloride |
| 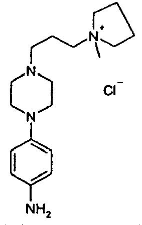 | 1-{3-[4-(4-Amino-phenyl)piperazin-1-yl]propyl}-1-methyl-pyrrolidinium chloride | | |
| 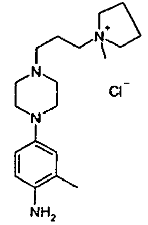 | 1-{3-[4-(4-Amino-3-methylphenyl)-piperazin-1-yl]-propyl}-1-methyl-pyrrolidinium chloride | | |

25. Nitro derivatives of formula (I') below 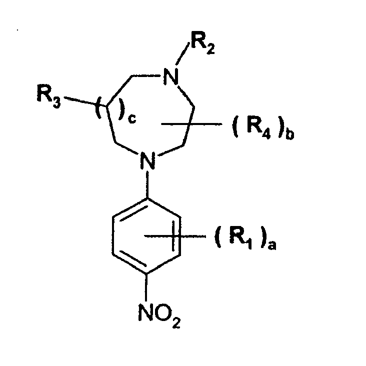 in which R₁, R₂, R₃, R₄, a, b and c are as defined according to any one of Claims 1 to 24.

26. Dye composition comprising, as oxidation base, at least one para-phenylenediamine derivative of formula (I) as defined according to any one of Claims 1 to 24.

27. Composition according to Claim 26, also comprising a coupler chosen from meta-phenylenediamines, meta-aminophenols, meta-diphenols, naphthalene-based couplers and heterocyclic couplers, and the addition salts thereof.

28. Composition according to either of Claims 26 and 27, comprising an additional oxidation base other than the oxidation bases of formula (I), chosen from para-phenylenediamines, bis(phenyl)alkylenediamines, para-aminophenols, ortho-aminophenols and heterocyclic bases, and the addition salts thereof.

29. Composition according to any one of Claims 26 to 28, in which the amount of each of the oxidation bases is between 0.001% and 10% by weight approximately relative to the total weight of the dye composition.

30. Composition according to Claim 29, in which the amount of each of the couplers is between 0.001% and 10% by weight approximately relative to the total weight of the dye composition.

31. Composition according to any one of Claims 26 to 30, comprising a cosmetic medium that is suitable for dyeing keratin fibres.

32. Process for the oxidation dyeing of keratin fibres, **characterized in that** a dye composition as defined in any one of Claims 26 to 31 is applied to the fibres in the presence of an oxidizing agent, for a time that is sufficient to develop the desired coloration.

33. Process according to Claim 32, in which the oxidizing agent is chosen from hydrogen peroxide, urea peroxide, alkali metal bromates, persalts, peracids and oxidase enzymes.

34. Multi-compartment device, in which a first compartment contains a dye composition as defined in any one of Claims 26 to 31 and a second compartment contains an oxidizing agent.

35. Use of the composition defined in Claims 26 to 31, for dyeing keratin fibres.

## Patentansprüche

1. *p*-Phenylendiaminderivat der Formel (I), das mit einer cyclischen Diazagruppe substituiert ist, sowie die entsprechenden Additionssalze: 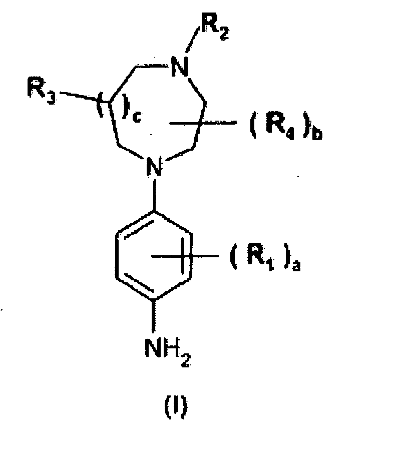 worin
• **a** im Bereich von 0 bis 4 liegt, mit der Maßgabe, dass die Gruppen R₁ gleich oder verschieden sein können, wenn a größer oder gleich 2 ist,
• **b** im Bereich von 0 bis 4 liegt, mit der Maßgabe, dass die Gruppen R₄ gleich oder verschieden sein können, wenn b größer oder gleich 2 ist,
• **c** 0 oder 1 bedeutet,
• **R**_{**1**} ein Halogenatom; eine aliphatische oder alicyclische, gesättigte oder ungesättigte C₁₋₈-Kohlenwasserstoffkette, wobei ein oder mehrere Kohlenstoffatome durch ein oder mehrere Sauerstoffatome, Stickstoffatome, Siliciumatome, Schwefelatome oder eine Gruppe SO₂ ersetzt sein können; eine Oniumgruppe Z bedeutet; wobei die Gruppe R₁ weder eine Peroxidbindung noch die Gruppen Diazo, Nitro oder Nitroso enthält,
• **R**_{**2**} eine Oniumgruppe Z bedeutet,
• **R**_{**3**} bedeutet:
- eine Alkylgruppe;
- eine Alkenylgruppe;
- eine Alkinylgruppe;
- eine Hydroxygruppe;
- eine Hydroxyalkylgruppe;
- eine Alkoxygruppe;
- eine Alkoxyalkylgruppe;
- eine Alkylcarbonylgruppe;
- eine Hydroxyalkoxyalkylgruppe;
- eine Aminogruppe,
- eine Monoalkylamino- oder Dialkylaminogruppe;
- eine Aminoalkylgruppe;
- eine Aminoalkylgruppe, bei der die Aminogruppe mit einer Alkylgruppe, Acetylgruppe oder Hydroxyalkylgruppe mono- oder disubstituiert ist;
- eine Hydroxygruppe und Aminoalkylgruppe;
- eine Carboxygruppe;
- eine Carboxyalkylgruppe;
- eine Carbamoylgruppe;
- eine Carbamoylalkylgruppe;
- eine Alkoxycarbonylgruppe;
- eine Mono- oder Dialkylaminocarbonylgruppe;
- eine Mono- oder Dialkylaminocarbonylalkylgruppe;
• **R**_{**4**} bedeutet:
- eine Alkylgruppe;
- eine Alkenylgruppe;
- eine Alkinylgruppe;
- eine Hydroxyalkylgruppe;
- eine Alkoxyalkylgruppe;
- eine Alkylcarbonylgruppe;
- eine Hydroxyalkoxyalkylgruppe;
- eine Aminoalkylgruppe,
- eine Aminoalkylgruppe, bei der die Aminogruppe mit einer Alkylgruppe, Acetylgruppe oder Hydroxyalkylgruppe mono- oder disubstituiert ist;
- eine Hydroxygruppe und Aminoalkylgruppe;
- eine Carboxygruppe;
- eine Carboxyalkylgruppe;
- eine Carbamoylgruppe;
- eine Carbamoylalkylgruppe;
- eine Alkoxycarbonylgruppe;
- eine Mono- oder Dialkylaminocarbonylgruppe;
- eine Mono- oder Dialkylaminocarbonylalkylgruppe.

2. Derivate nach Anspruch 1, wobei R₁ unter C₁₋₄-Alkyl, C₁₋₄-Alkoxyalkyl, C₁₋₄-Hydroxyalkyl, C₁₋₄-Aminoalkyl, C₁₋₄-Alkoxy, C₁₋₄-Hydroxyalkoxy und C₁₋₄-Carboxyalkyl ausgewählt ist.

3. Derivate nach Anspruch 2, worin R₁ unter Methyl, Hydroxymethyl, 2-Hydroxyethyl, 1,2-Dihydroxyethyl, Methoxy, Isopropyloxy und 2-Hydroxyethoxy ausgewählt ist.

4. Derivate nach Anspruch 1, 2 oder 3, wobei **a** 0 oder 1 bedeutet.

5. Derivate nach einem der Ansprüche 1 bis 4, wobei R₂ der Oniumgruppe Z der folgenden Formel (II) entspricht: 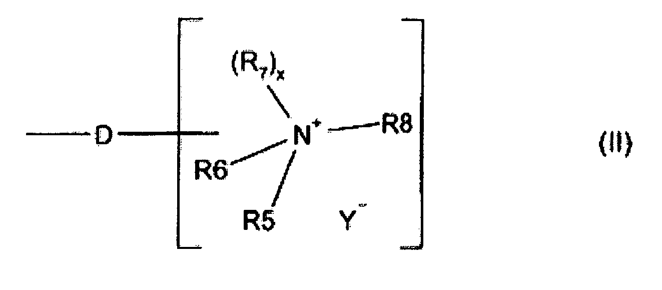 worin bedeuten:
- D eine kovalente Bindung oder eine geradkettige oder verzweigte C₁₋₁₄-Alkylenkette, die ein oder mehrere Heteroatome enthalten kann, die unter Sauerstoff, Schwefel oder Stickstoff ausgewählt sind, und mit einer oder mehreren Hydroxygruppen oder Aminogruppen substituiert sein kann und eine oder mehrere Carbonylgruppen enthalten kann,
- **R**_{**8**}**, R**_{**5**} und **R**_{**6**} unabhängig voneinander C₁₋₁₅-Alkyl, C₁₋₆-Monohydroxyalkyl; C₂₋₆-Polyhydroxyalkyl; Alkoxy(C₁₋₆)-alkyl(C₁₋₆); Aryl; Benzyl; Carbamoylalkyl(C₁₋₆); Trialkyl(C₁₋₆)-silanalkyl(C₁₋₆); C₁₋₆-Aminoalkyl; C₁₋₆-Aminoalkyl, worin die Aminogruppe mono- oder disubstituiert ist mit C₁₋₄-Alkyl, Alkyl(C₁₋₆)carbonyl, Carbamoyl oder Alkyl(C₁₋₆)sulfonyl; eine quartäre Ammoniumgruppe;
- **R**_{**8**}**, R**_{**5**} und **R**_{**6**} gemeinsam, paarweise, mit dem Stickstoffatom, an das sie gebunden sind, einen 4-, 5-, 6- oder 7-gliedrigen gesättigten Kohlenstoffring, der ein oder mehrere Heteroatome enthalten kann, wobei der Ring substituiert sein kann mit Hydroxy, C₁₋₆-Alkyl, C₁₋₆-Monohydroxyalkyl; C₂₋₆-Polyhydroxyalkyl; C₁₋₆-Alkoxy, Trialkyl(C₁₋₆)silanalkyl-(C₁₋₆); Carbamoyl, Carboxyl, Alkyl(C₁₋₆)carbonyl, Amino, einer Aminogruppe, die mono- oder disubstituiert ist mit (C₁₋₆)-Alkyl, Alkyl(C₁₋₆)carbonyl, Carbamoyl oder Alkyl(C₁₋₆)-sulfonyl;
- **R**_{**7**} C₁₋₆-Alkyl; C₁₋₆-Monohydroxyalkyl; C₂₋₆-Polyhydroxyalkyl; Aryl; Benzyl; C₁₋₆-Aminoalkyl; C₁₋₆-Aminoalkyl, worin die Aminogruppe mono- oder disubstituiert ist mit Alkyl(C₁₋₆), Alkyl-(C₁₋₆)carbonyl, Carbamoyl oder Alkyl(C₁₋₆)sulfonyl; Carboxyalkyl(C₁₋₆); Carbamoylalkyl(C₁₋₆); Trifluoralkyl(C₁₋₆); Trialkyl-(C₁₋₆)silanalkyl(C₁₋₆); Sulfonamidoalkyl(C₁₋₆); Alkyl(C₁₋₆)-carboxyalkyl(C₁₋₆); Alkyl(C₁₋₆)sulfinylalkyl(C₁₋₆); Alkyl(C₁₋₆)-sulfonylalkyl(C₁₋₆); Alkyl(C₁₋₆)carbonylalkyl(C₁₋₆); N-Alkyl-(C₁₋₆)carbamoyl(C₁₋₆); N-Alkyl(C₁₋₆)sulfonamidoalkyl(C₁₋₆);
• x 0 oder 1,
- wenn x = 0, ist die Verbindungsgruppe an das Stickstoffatom gebunden, das die Gruppen R₅, R₆ und R₈ trägt,
- wenn x = 1, bilden zwei der Gruppen R₅, R₆ und R₈ gemeinsam mit dem Stickstoffatom, an das sie gebunden sind, einen 4-, 5-, 6- oder 7-gliedrigen gesättigten Ring und D ist an ein Kohlenstoffatom des gesättigten Rings gebunden;
• Y⁻ ein Gegenion.

6. Derivate nach Anspruch 5, worin x 0 bedeutet und R₅, R₆ und R₈ unabhängig voneinander ausgewählt sind unter C₁₋₆-Alkyl, C₁₋₄-Monohydroxyalkyl, C₂₋₄-Polyhydroxyalkyl, Alkoxy(C₁₋₆)-alkyl(C₁₋₄), Carbamoylalkyl(C₁₋₆), Trialkyl(C₁₋₆)silanalkyl(C₁₋₆), oder R₈ bildet gemeinsam mit R₅ einen Azetidinring, Pyrrolidinring, Piperidinring, Piperazinring oder Morpholinring, wobei R₆ in diesem Fall unter C₁₋₆-Alkyl; C₁₋₆-Monohydroxyalkyl; C₂₋₆-Polyhydroxyalkyl; C₁₋₆-Aminoalkyl, einer Aminoalkylgruppe, die mono- oder disubstituiert ist mit Alkyl(C₁₋₆), Alkyl(C₁₋₆)carbonyl, Carbamoyl oder Alkyl(C₁₋₆)sulfonyl; Carbamoylalkyl(C₁₋₆); Trialkyl(C₁₋₆)silanalkyl(C₁₋₆); Alkyl(C₁₋₆)carboxyalkyl(C₁₋₆); Alkyl(C₁₋₆)carbonylalkyl(C₁₋₆); N-Alkyl(C₁₋₆)carbamoylalkyl(C₁₋₆) ausgewählt ist.

7. Derivate nach Anspruch 5, worin x 1 bedeutet, R₇ ausgewählt ist unter C₁₋₆-Alkyl; C₁₋₆-Monohydroxyalkyl; C₂₋₆-Polyhydroxyalkyl; C₁₋₆-Aminoalkyl; C₁₋₆-Aminoalkyl, worin die Aminogruppe mono- oder disubstituiert ist mit Alkyl(C₁₋₆), Alkyl(C₁₋₆)carbonyl, Carbamoyl oder Alkyl(C₁₋₆)sulfonyl; Carbamoylalkyl(C₁₋₆); Trialkyl-(C₁₋₆)silanalkyl(C₁₋₆); Alkyl(C₁₋₆)carboxyalkyl(C₁₋₆); Alkyl(C₁₋₆)-carbonylalkyl(C₁₋₆); N-Alkyl(C₁₋₆)carbamoylalkyl(C₁₋₆); R₈ gemeinsam mit R₅ einen Azetidinring, Pyrrolidinring, Piperidinring, Piperazinring oder Morpholinring bildet, wobei R₆ in diesem Fall unter C₁₋₆-Alkyl; C₁₋₆-Monohydroxyalkyl; C₂₋₆-Polyhydroxyalkyl; C₁₋₆-Aminoalkyl, C₁₋₆-Aminoalkyl, worin die Aminogruppe mono- oder disubstituiert ist mit Alkyl(C₁₋₆), Alkyl(C₁₋₆)carbonyl, Carbamoyl oder Alkyl(C₁₋₆)sulfonyl; Carbamoylalkyl(C₁₋₆); Trialkyl(C₁₋₆)-silanalkyl(C₁₋₆); Alkyl(C₁₋₆)carboxyalkyl(C₁₋₆); Alkyl(C₁₋₆)carbonylalkyl(C₁₋₆); N-Alkyl(C₁₋₆)carbamoylalkyl(C₁₋₆).

8. Derivate nach Anspruch 5, worin R₂ eine Trialkylammoniumalkylgruppe bedeutet, wobei die Alkylgruppe, die R₂ mit dem Ring verbindet, mit einer oder mehreren Hydroxygruppen substituiert sein kann.

9. Derivate nach Anspruch 5, worin D eine kovalente Bindung oder eine Alkylenkette bedeutet, die substituiert sein kann und/oder eine Carbonylgruppe enthalten kann.

10. *p*-Phenylendiaminderivate nach einem der Ansprüche 1 bis 4, worin R₂ eine Oniumgruppe Z der folgenden Formel (III) bedeutet: 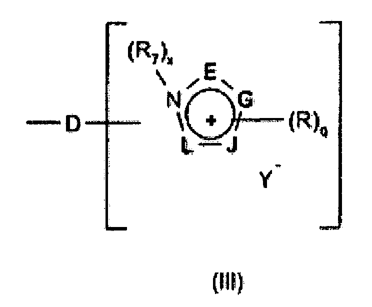 worin bedeuten
• D eine kovalente Bindung oder eine geradkettige oder verzweigte C₁₋₁₄-Alkylenkette, die ein oder mehrere Heteroatome enthalten kann, die unter Sauerstoff, Schwefel oder Stickstoff ausgewählt sind, und mit einer oder mehreren Hydroxygruppen oder Aminogruppen substituiert sein kann und eine oder mehrere Carbonylgruppen enthalten kann;
• die Ringatome E, G, J und L, die gleich oder verschieden sind, ein Kohlenstoffatom, ein Sauerstoffatom, ein Schwefelatom oder ein Stickstoffatom zur Bildung eines Pyrrolrings, eines Pyrazolrings, Imidazolrings, Triazolrings, Oxazolrings, Isooxazolrings, Thiazolrings oder Isothiazolrings,
• q eine ganze Zahl im Bereich von 1 bis 4;
• die Gruppen **R,** die gleich oder verschieden sind, ein Wasserstoffatom, ein Halogenatom, eine Hydroxygruppe, C₁₋₆-Alkyl, C₁₋₆-Monohydroxyalkyl, C₂₋₆-Polyhydroxyalkyl, C₁₋₆-Alkoxy, Trialkyl(C₁₋₆)silanalkyl(C₁₋₆), Carbamoyl, Carboxy, C₁₋₆-Alkylcarbonyl; Amino, eine Aminogruppe, die mono- oder disubstituiert ist mit Alkyl(C₁₋₆), Alkyl(C₁₋₆)carbonyl, Carbamoyl oder Alkyl(C₁₋₆)sulfonyl; C₁₋₆-Monohydroxyalkyl, C₂₋₆-Polyhydroxyalkyl oder eine quartäre Ammoniumgruppe;
• **R**_{**7**} C₁₋₆-Alkyl; C₁₋₆-Monohydroxyalkyl; C₂₋₆-Polyhydroxyalkyl; Aryl; Benzyl; C₁₋₆-Aminoalkyl; C₁₋₆-Aminoalkyl, worin die Aminogruppe mit Alkyl(C₁₋₆), Alkyl(C₁₋₆)carbonyl, Carbamoyl oder Alkyl(C₁₋₆)sulfonyl substituiert ist; C₁₋₆-Carboxyalkyl; Carbamoylalkyl(C₁₋₆); Trifluoralkyl(C₁₋₆); Trialkyl(C₁₋₆)silanalkyl(C₁₋₆); Sulfonamidoalkyl(C₁₋₆); Alkyl(C₁₋₆)carboxyalkyl(C₁₋₆); Alkyl-(C₁₋₆)sulfinylalkyl(C₁₋₆); Alkyl(C₁₋₆)sulfonylalkyl(C₁₋₆); Alkyl-(C₁₋₆)carboxyalkyl(C₁₋₆); N-Alkyl(C₁₋₆)carbamoylalkyl(C₁₋₆); N-Alkyl(C₁₋₆)sulfonamidoalkyl(C₁₋₆); eine quartäre Ammoniumgruppe;
• x 0 oder 1,
- wenn x = 0, ist die Verbindungsgruppe D an das Stickstoffatom gebunden,
- wenn x = 1, ist die Verbindungsgruppe D an ein Ringatom E, G, J oder L gebunden,
• Y⁻ ein Gegenion.

11. Derivate nach Anspruch 10, worin die Ringatome E, G, J und L einen Pyrrolring, Imidazolring, Pyrazolring, Oxazolring, Thiazolring oder Triazolring bilden.

12. Derivate nach Anspruch 11, worin die Ringatome E, G, J und L einen Imidazolring bilden.

13. Derivate nach einem der Ansprüche 10 bis 12, worin x 0 ist, D eine kovalente Bindung bedeutet oder eine Alkylenkette, die substituiert sein kann und/oder eine Carbonylfunktion enthalten kann.

14. *p*-Phenylendiaminderivate nach einem der Ansprüche 1 bis 4, worin R₂ eine Oniumgruppe Z der folgenden Formel (IV) bedeutet: 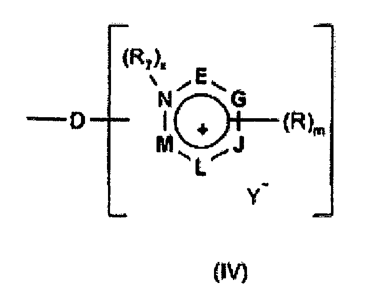 worin bedeuten:
• D eine kovalente Bindung oder eine geradkettige oder verzweigte C₁₋₁₄-Alkylenkette, die ein oder mehrere Heteroatome enthalten kann, die unter Sauerstoff, Schwefel oder Stickstoff ausgewählt sind, und mit einer oder mehreren Hydroxygruppen oder Aminogruppen substituiert sein kann und eine oder mehrere Carbonylgruppen enthalten kann;
• die Ringatome E, G, J, L und M, die gleich oder verschieden sind, ein Kohlenstoffatom, Sauerstoffatom, Schwefelatom oder Stickstoffatom, wobei sie einen Ring bilden, der unter den Ringen Pyridin, Pyrimidin, Pyrazin, Triazin, Pyridazin ausgewählt ist,
• **m** eine ganze Zahl von 1 bis 5;
• die Gruppen **R,** die gleich oder verschieden sind, ein Wasserstoffatom, ein Halogenatom, eine Hydroxygruppe, C₁₋₆-Alkyl, C₁₋₆-Monohydroxyalkyl, C₂₋₆-Polyhydroxyalkyl, C₁₋₆-Alkoxy; Trialkyl(C₁₋₆)silanalkyl(C₁₋₆); Carbamoyl, Carboxy, Alkylcarbonyl(C₁₋₆); Amino, eine Aminogruppe, die substituiert ist mit Alkyl(C₁₋₆), Alkyl(C₁₋₆)carbonyl, Carbamoyl oder Alkyl(C₁₋₆)-sulfonyl; C₁₋₆-Monohydroxyalkyl, C₂₋₆-Polyhydroxyalkyl oder eine quartäre Ammoniumgruppe;
• **R**_{**7**} C₁₋₆-Alkyl; C₁₋₆-Monohydroxyalkyl; C₂₋₆-Polyhydroxyalkyl; Aryl; Benzyl; C₁₋₆-Aminoalkyl; C₁₋₆-Aminoalkyl, worin die Aminogruppe mit Alkyl(C₁₋₆), Alkyl(C₁₋₆)carbonyl, Carbamoyl oder Alkyl(C₁₋₆)sulfonyl mono- oder disubstituiert ist; C₁₋₆-Carboxyalkyl; Carbamoylalkyl(C₁₋₆); Trifluoralkyl(C₁₋₆); Trialkyl(C₁₋₆)-silanalkyl(C₁₋₆); Sulfonamidoalkyl(C₁₋₆); Alkyl(C₁₋₆)carboxyalkyl(C₁₋₆); Alkyl(C₁₋₆)sulfinylalkyl(C₁₋₆); Alkyl(C₁₋₆)sulfonylalkyl(C₁₋₆); Alkyl(C₁₋₆)carboxyalkyl(C₁₋₆); N-Alkyl(C₁₋₆)-carbamoylalkyl(C₁₋₆); N-Alkyl(C₁₋₆)sulfonamidoalkyl(C₁₋₆); eine quartäre Ammoniumgruppe;
• x 0 oder 1,
- wenn x = 0, ist die Verbindungsgruppe D an das Stickstoffatom gebunden,
- wenn x = 1, ist die Verbindungsgruppe D an ein Ringatom E, G, J, L oder M gebunden.,
• Y⁻ ein Gegenion.

15. Derivate nach Anspruch 14, wobei die Ringbestandteile E, G, J, L und M mit dem Stickstoffatom des Rings einen Ring bilden, der unter den Ringen Pyridin und Pyrimidin ausgewählt ist.

16. Derivate nach einem der Ansprüche 10 bis 15, worin x 0 bedeutet und **R** unter Wasserstoff, Hydroxy, C₁₋₆-Alkyl, C₁₋₆-Monohydroxyalkyl, C₂₋₆-Polyhydroxyalkyl, C₁₋₆₋Alkoxy; Trialkyl(C₁₋₆)-silanalkyl(C₁₋₆); Carbamoyl, Alkylcarbonyl(C₁₋₆), Amino, einer Aminogruppe, die mono- oder disubstituiert ist mit Alkyl(C₁₋₆), Alkyl(C₁₋₆)carbonyl, Carbamoyl oder Alkyl(C₁₋₆)sulfonyl; C₁₋₆-Monohydroxyalkyl oder C₂₋₆-Polyhydroxyalkyl ausgewählt ist.

17. Derivate nach einem der Ansprüche 10 bis 15, worin x 1 bedeutet, **R**_{**7**} ausgewählt ist unter C₁₋₆-Alkyl; C₁₋₆-Monohydroxyalkyl; C₂₋₆-Polyhydroxyalkyl; C₁₋₆-Aminoalkyl; C₁₋₆-Aminoalkyl, worin die Aminogruppe mono- oder disubstituiert ist mit Alkyl(C₁₋₆), Alkyl(C₁₋₆)carbonyl, Carbamoyl oder Alkyl(C₁₋₆)sulfonyl; Carbamoylalkyl(C₁₋₆); Trialkyl(C₁₋₆)silanalkyl(C₁₋₆); Alkyl(C₁₋₆)carbonylalkyl(C₁₋₆); Alkyl(C₁₋₆)carbamoyl; und **R** ausgewählt ist unter Wasserstoff, Hydroxy, C₁₋₆-Alkyl, C₁₋₆-Monohydroxyalkyl, C₂₋₆-Polyhydroxyalkyl, C₁₋₆-Alkoxy, Trialkyl(C₁₋₆)silanalkyl(C₁₋₆), Carbamoyl, C₁₋₆-Alkylcarbonyl, Amino, einer Aminogruppe, die mono- oder disubstituiert ist mit Alkyl(C₁₋₆), Alkyl(C₁₋₆)carbonyl, Carbamoyl oder Alkyl(C₁₋₆)sulfonyl.

18. Derivate nach einem der Ansprüche 10 bis 17, worin **R** ausgewählt ist unter Wasserstoff oder Alkylgruppen, die substituiert sein können, und R₇ eine Alkylgruppe bedeutet, die substituiert sein kann.

19. Derivate nach einem der Ansprüche 1 bis 18, worin **R** ausgewählt ist unter Wasserstoff; Alkyl; einer Alkylgruppe, die mit einer oder mehreren Hydroxygruppen substituiert ist; einer Alkylgruppe, die mit einer oder mehreren Aminogruppen substituiert ist; Carboxy, Carbamoyl, Amino, Hydroxy.

20. Derivate nach Anspruch 19, worin **R** ausgewählt ist unter Wasserstoff; Hydroxy; Methyl; Amino; Hydroxymethyl; Aminomethyl.

21. Derivate nach einem der Ansprüche 1 bis 20 mit b=0 oder worin **R**_{**4**} ausgewählt ist unter einer Alkylgruppe; einer Alkylgruppe, die mit einer oder mehreren Hydroxygruppen substituiert ist; einer Alkylgruppe, die mit einer oder mehreren Aminogruppen substituiert ist; Carboxy; Carbamoyl.

22. Derivate nach einem der vorhergehenden Ansprüche, worin **R**_{**3**} ausgewählt ist unter Wasserstoff; Hydroxy; Amino, Alkyl; einer Alkylgruppe, die mit einer oder mehreren Hydroxygruppen substituiert ist; einer Alkylgruppe, die mit einer oder mehreren Aminogruppen substituiert ist; Carboxy; Carbamoyl.

23. Derivate nach einem der Ansprüche 1 bis 22, wobei das Gegenion Y⁻ ausgewählt ist unter einem Halogenatom, Hydroxid, Hydrogensulfat, Acetat, Tartrat oder einem Alkyl(C₁₋₆)sulfat.

24. Derivate nach einem der vorhergehenden Ansprüche, die unter den folgenden Verbindungen ausgewählt sind:
| | | | |
|---|---|---|---|
| 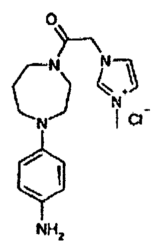 | 3-{2-[4-(4-Amino-phényl)-[1,4]diazepan-1-yl]-2-oxo-ethyl}-1-méthyl-3H-imidazol-1-ium-chlorid | 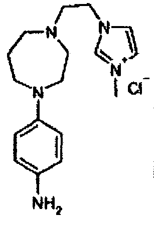 | 3-{2-[4-(4-Amino-phenyl)-[1,4]diazèpan-1-yl]-éthyl}-1-méthyl-3H-imidazol-1-ium-chlorid |
| 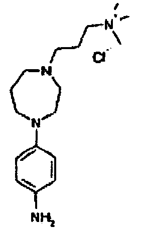 | {3-[4-(4-Amino-phenyl)-[1,4]diazépan-1-yl]-propyl}-trimethyl-ammonium-chlorid | 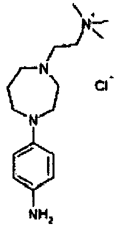 | {2-[4-(4-Amino-phényl)-[1,4]diazepan-1-yl]-éthyl}-trimethyl-ammonium-chlorid |
| 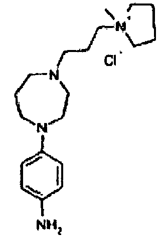 | 1-{3-[4-(4-Amino-phényl)-[1,4]diazepan-1-yl]-propyl)-1 -methyl-pyrrolidinium-chlorid | 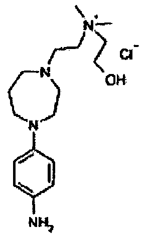 | {2-[4-(4-Amino-phenyl)-[1,4]diazepan-1-yl]-éthyl)-(2-hydroxy-ethyl)-diméthyl-ammoniumchlorid |
| 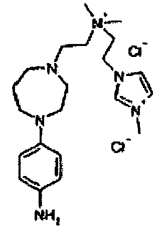 | {3-[4-(4-Amino-phenyl)-[1,4]diazepan-1-yl]-éthyl}-dimethyl(ethyl-2-methyl-3H-imidazol-1-ium -chlorid ) -ammoniumchlorid | 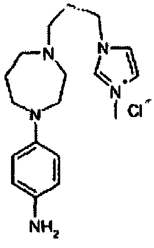 | 3-{2-[4-(4-Amino-phenyl)-[1,4]diazepan-1-yl]-propyl}-1-méthyl-3H-imidazol-1-ium-chlorid |
| 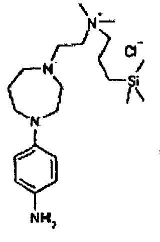 | {2-[4-(4-Amino-phenyl)-[1,4]diazepan-1-yl]-éthyl}-dimethyl-(3-trimethylsilanyl-propyl)-ammonium-chlorid | 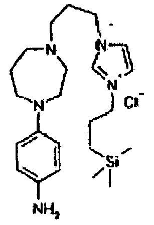 | 3-{2-[4-(4-Amino-phenyl)-[1,4]diazepan-1-yl]-éthyl)-1-(3-trimethylsilanyl-propyl)-3H-imidazol-1-ium -chlorid |
| 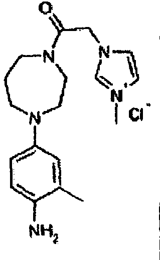 | 3-{2-[4-(4-Amino-3-methyl-phenyl)-[1,4]diazepan-1-yl]-2-oxo-ethyl}-1-methyl-3H-imidazol-1-ium-chlorid | 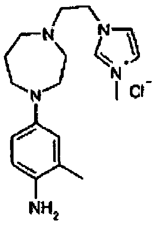 | 3-{2-[4-(4-Amino-3-methyl-phenyl)-[1,4]diazepan-1-yl]-ethyl}-1-melhyl-3H-imitlazol-1-ium-chlorid |
| 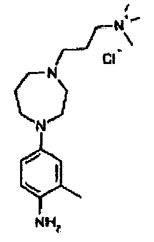 | {3-[4-(4-Amino-3-methyl-phényl)-[1,4]diazepan-1-yl]-propyl}-trimethyl-ammonium-chlorid | 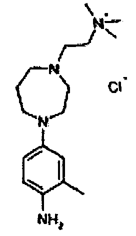 | {2-[4-(4-Amino-3-méthyl-phenyl)-[1,4]diazepan-1-yl]-éthyl}-trimethyl-ammonium-chlorid |
| 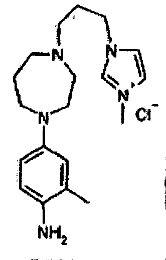 | 3-{2-[4-(4-Amino-3-methyl-phényl)-[1,4]diazepan-1-yl]-propyl)-1-méthyl-3H-imidazol-1-ium-chlorid | 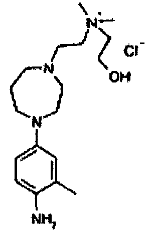 | {2-[4-(4-Amino-3-methyl-phenyl)-[1,4]diazepan-1-yl]-èthyl}-(2-hydroxy-éthyl)-dimethyl-ammonium-chlorid |
| 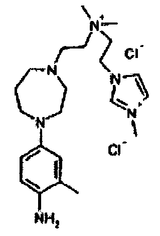 | {3-[4-(4-Amino-3-methyl-phényl)-[1,4]diazepan-1-yl]-éthyl}-diméthyl(éthyl-2--methyl-3H-imidazol-1-ium chlorure)-ammonium-chlorid | 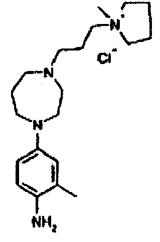 | 1-(3-[4-(4-Amino-3-méthyl-phenyl)-[1,4]diazepan-1-yl]-propyl}-1-methyl-pyrrolidinium -chlorid |
| 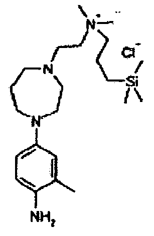 | {2-[4-(4-Amino-3-methyl-phényl)-(1,4)diazépan-1-yl]-ethyl}-diméthyl-(3-trimethylsilanyl-propyl)-ammonium-chlorid | 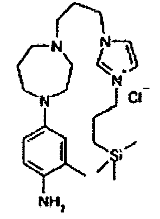 | 3-{2-[4-(4-Amino-3-methyl-phényl)-[1,4]diazepan-1-yl]-ethyl}-1-(3-trimethylsilanyl-propyl)-3H-imidazol-1-ium-chlorid |
| 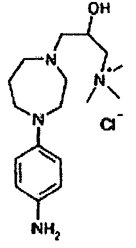 | {3-[4-(4-Amino-phenyl)-[1,4]diazepan-1-yl]-2-hydroxy-propyl}-trimethyl-ammonium-chlorid | 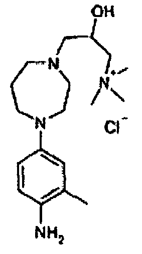 | {3-[4-(4-Amino-3-methyl-phényl)-[1,4]diazépan-1-yl]-2-hydroxy-propyl)-trimethyl-ammonium-chlorid |
| 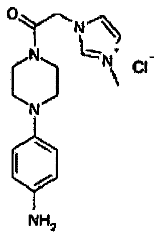 | 3-{2-[4-(4-Amino-phenyl)-piperazin-1-yl]-2-oxo-ethyl)-1-methyl-3H-imidazol-1-ium-chlorid | 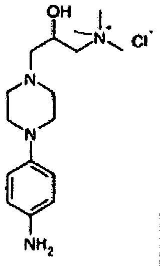 | {3-[4-(4-Amino-phenyl)-piperazin-1-yl]-2-hydroxy-propyl}-triméthyl-ammonium-chlorid |
| 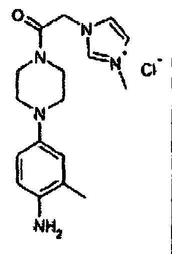 | 3-{2-[4-(4-Amino-3-methyl-phenyl)-piperazin-1-yl]-2-oxo-ethyl}-1-methyll-3H-imidazol-1-ium-chlorid | 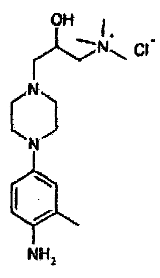 | 3-[4-(4-Amino-3-methyl-phenyl)-piperazin-1-yl]-2-hydroxy-propyl}-trimethyl-ammonium -chlorid |
| 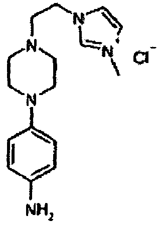 | 3-{2-[4-(4-Amino-phenyl)-piperazin-1-yl]-ethyl}-1-methyl-3H-imidazol-1-ium-chlorid | 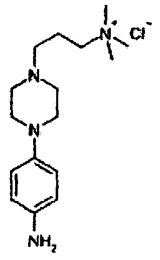 | {3-[4-(4-Amino-phényl)-piperazin-1-yl]-propyl}-trimethyl-ammonium-chlorid |
| 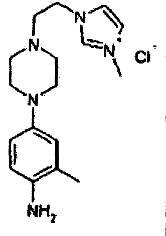 | 3-(2-[4-(4-Amino-3-methyl-phenyl)-piperazin-1-yl]-ethyl}-1-methyl-3H-imidazol-1-ium-chlorid | 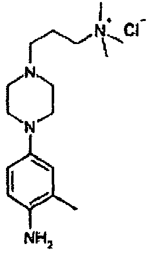 | {3-[4-(4-Amino-3-methyl-phenyl)-piperazin-1-yl]-propyl}-triméthyl-ammonium-chlorid |
| 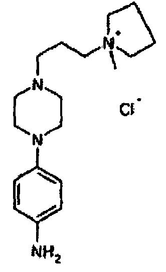 | 1-{3-[4-(4-Amino-phenyl)-piperazin-1-yl]-propyl}-1-méthyl-pyrrolidinium-chlorid | | |
| 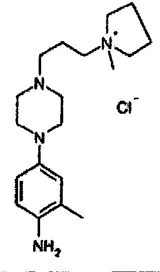 | 1-{3-[4-(4-Amino-3-méthyl-phényl)-piperazin-1-yl]-propyl}-1-methyl-pyrrolidinium-chlorid | | |

25. Nitroderivate der folgenden Formel (I') 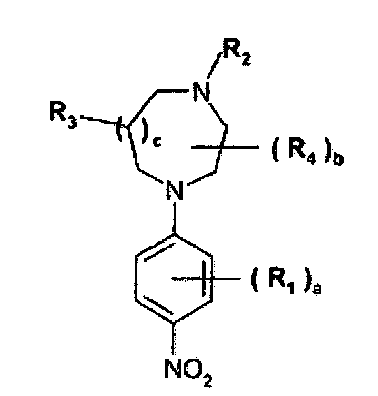 worin die Gruppen R₁, R₂, R₃, R₄, a, b, c die in einem der Ansprüche 1 bis 24 angegebenen Bedeutungen aufweisen.

26. Farbmittelzusammensetzung, die als Oxidationsbase mindestens ein *p*-Phenylendiaminderivat der Formel (I) nach einem der Ansprüche 1 bis 24 enthält.

27. Zusammensetzung nach Anspruch 26, die ferner einen Kuppler enthält, der unter den *m*-Phenylendiaminen, *m*-Aminophenolen, *m*-Dihydroxybenzolen, Naphthalinkupplern, heterocyclischen Kupplern und deren Additionssalzen ausgewählt ist.

28. Zusammensetzung nach einem der Ansprüche 26 oder 27, die eine zusätzliche Oxidationsbase enthält, die von den Oxidationsbasen der Formel (I) verschieden ist und unter den *p*-Phenylendiaminen, Bisphenylalkylendiaminen, p-Aminophenolen, o-Aminophenolen, heterocyclischen Basen und deren Additionssalzen ausgewählt ist.

29. Zusammensetzung nach einem der Ansprüche 26 bis 28, worin der Mengenanteil jeder Oxidationsbase im Bereich von etwa 0,001 bis 10 Gew.-% des Gesamtgewichts der Farbmittelzusammensetzung liegt.

30. Zusammensetzung nach Anspruch 29, worin der Mengenanteil jedes Kupplers im Bereich von etwa 0,001 bis 10 Gew.-% des Gesamtgewichts der Farbmittelzusammensetzung liegt.

31. Zusammensetzung nach einem der Ansprüche 26 bis 30, die ein zum Färben von Keratinfasern geeignetes kosmetisches Medium enthält.

32. Verfahren zum oxidativen Färben von Keratinfasern, **dadurch gekennzeichnet, dass** auf die Fasern eine Farbmittelzusammensetzung nach einem der Ansprüche 26 bis 31 in Gegenwart eines Oxidationsmittels während einer Zeitspanne aufgebracht wird, die ausreichend ist, um die gewünschte Färbung zu bilden.

33. Verfahren nach Anspruch 32, worin das Oxidationsmittel unter Wasserstoffperoxid, Harnstoffperoxid, Alkalimetallbromaten, Salzen von Persäuren, Persäuren und Oxidasen ausgewählt ist.

34. Vorrichtung mit mehreren Abteilungen, worin eine erste Abteilung eine Farbmittelzusammensetzung nach einem der Ansprüche 26 bis 31 und eine zweite Abteilung ein Oxidationsmittel enthält.

35. Verwendung der Zusammensetzung nach einem der Ansprüche 26 bis 31 zum Färben von Keratinfasern.
